# EUROPEAN PATENT SPECIFICATION

(11) **EP 1 411 979 B1**
(45) Date of publication and mention of the grant of the patent: **06.04.2011**
(21) Application number: 02791473.8
(22) Date of filing: 26.07.2002
(51) Int. Cl.: A61K 39/21, A61K 31/713, C12N 15/49, C12N 15/89, C07K 14/16, C07K 19/00

(54) **VACCINE COMPRISING GP120 AND NEF AND/OR TAT FOR THE IMMUNISATION AGAINST HIV**
VAKZINE, WELCHE GP120 ZUSAMMEN MIT NEF UND/ODER TAT ENTHÄLT, ZUR IMMUNISIERUNG GEGEN HIV
VACCINE COMPRENANT GP120 ET NEF ET/OU TAT POUR L'IMMUNISATION CONTRE HIV

(30) Priority: 27.07.2001 GB 0118367
(43) Date of publication of application: 28.04.2004
(73) Proprietor: GlaxoSmithKline Biologicals s.a., 1330 Rixensart (BE); GLAXO GROUP LIMITED, Greenford, Middlesex UB6 0NN (GB)
(72) Inventor: ERTL, Peter Franz, Stevenage, Hertfordshire SG1 2NY (GB); TITE, John Philip, Stevenage, Hertfordshire SG1 2NY (GB); VAN WELY, Catherine Ann, Stevenage, Hertfordshire SG1 2NY (GB); VOSS, Gerald, GlaxoSmithKline Biologicals, 1330 Rixensart (BE)
(74) Representative: Hambleton, Bernadette Angelina
(86) International application number: PCT/EP2002/008343
(87) International publication number: WO 2003/011334

(56) References cited:
- WO-A-01/04280
- WO-A-01/54719
- WO-A-99/16884
- HEYDENBURG FULLER D ET AL: "A QUALITATIVE PROGRESSION IN HIV TYPE 1 GLYCOPROTEIN 120-SPECIFIC CYTOTOXIC CELLULAR AND HUMORAL IMMUNE RESPONSES IN MICE RECEIVING ADNA-BASED GLYCOPROTEIN 120 VACCINE" AIDS RESEARCH AND HUMAN RETROVIRUSES, NEW YORK, NY, US, vol. 10, no. 11, 1 November 1994 (1994-11-01), pages 1433-1441, XP000572444 ISSN: 0889-2229
- COLLINGS A ET AL: "Humoral and cellular immune responses to HIV-1 Nef in mice DNA-immunised with non-replicating or self-replicating expression vectors" VACCINE, BUTTERWORTH SCIENTIFIC. GUILDFORD, GB, vol. 18, 2000, pages 460-467, XP002954558 ISSN: 0264-410X
- CIERNIK I F ET AL: "INDUCTION OF CYTOTOXIC T LYMPHOCYTES AND ANTITUMOR IMMUNITY WITH DNA VACCINES EXPRESSING SINGLE T CELL EPITOPES" JOURNAL OF IMMUNOLOGY, THE WILLIAMS AND WILKINS CO. BALTIMORE, US, vol. 156, no. 7, 1 April 1996 (1996-04-01), pages 2369-2375, XP002024721 ISSN: 0022-1767
- GAHERY-SEGARD H ET AL: "Multiepitopic B- and T-cell response induced in humans by a human immunodeficiency virus type 1 lipopeptide vaccine" JOURNAL OF VIROLOGY, THE AMERICAN SOCIETY FOR MICROBIOLOGY, US, vol. 74, no. 4, February 2000 (2000-02), pages 1694-1703, XP002178429 ISSN: 0022-538X
- EVANS T G ET AL: "A canarypox vaccine expressing multiple human immunodeficiency virus type 1 genes given alone or with Rgp120 elicits broad and durable CD8+ cytotoxic T lymphocyte responses in seronegative volunteers" JOURNAL OF INFECTIOUS DISEASES, CHICAGO, IL, US, vol. 180, no. 2, August 1999 (1999-08), pages 290-298, XP002176373 ISSN: 0022-1899
- AGWALE S M ET AL: "A Tat subunit vaccine confers protective immunity against the immune-modulating activity of the human immunodeficiency virus type-1 Tat protein in mice." PROCEEDINGS OF THE NATIONAL ACADEMY OF SCIENCES OF THE UNITED STATES OF AMERICA. UNITED STATES 23 JUL 2002, vol. 99, no. 15, 23 July 2002 (2002-07-23), pages 10037-10041, XP002226223 ISSN: 0027-8424

## Description

The present invention relates to novel uses of HIV proteins in medicine and vaccine compositions containing such HIV proteins. In particular, the invention relates to the use of HIV Tat and HIV gp120 proteins in combination Furthermore, the invention relates to the use of HIV Nef and gp 120 proteins in combination. The invention also relates to DNA encoding HIV Tat and/or Nef (hereinafter Tat DNA and/or Nef DNA) and DNA encoding HIV gp120 (hereinafter gp120 DNA) and vectors comprising such DNAs. The invention relates in particular to administering the proteins and/or DNAs in a prime-boost schedule via a particle bombardment approach.

HIV-1 is the primary cause of the acquired immune deficiency syndrome (AIDS) which is regarded as one of the world's major health problems. Although extensive research throughout the world has been conducted to produce a vaccine, such efforts thus far have not been successful.

The HIV envelope glycoprotein gp120 is the viral protein that is used for attachment to the host cell. This attachment is mediated by the binding to two surface molecules of helper T cells and macrophages, known as CD4 and one of the two chemokine receptors CCR-4 or CXCR-5. The gp 120 protein is first expressed as a larger precursor molecule (gp160), which is then cleaved post-translationally to yield gp120 and gp41. The gp120 protein is retained on the surface of the virion by linkage to the gp41 molecule, which is inserted into the viral membrane.

The gp120 protein is the principal target of neutralizing antibodies, but unfortunately the most immunogenic regions of the proteins (V3 loop) are also the most variable parts of the protein. Therefore, the use of gp120 (or its precursor gp160) as a vaccine antigen to elicit neutralizing antibodies is thought to be of limited use for a broadly protective vaccine. The gp120 protein does also contain epitopes that are recognized by cytotoxic T lymphocytes (CTL). These effector cells are able to eliminate virus-infected cells, and therefore constitute a second major antiviral immune mechanism. In contrast to the target regions of neutralizing antibodies some CTL epitopes appear to be relatively conserved among different HIV strains. For this reason gp120 and gp160 are considered to be useful antigenie components in vaccines that aim at eliciting cell-mediated immune responses (particularly CTL).

Non-envelope proteins of HIV-1 have been described and include for example internal structural proteins such as the products of the *gag* and *pol* genes and, other non-structural proteins such as Rev, Nef, Vif and Tat (Greene et al., New England J. Med, 324, 5, 308 et seq (1991) and Bryant et al. (Ed. Pizzo), Pediatr. Infect. Dis. J., 11, 5, 390 et seq (1992).

HIV Tat and Nef proteins are early proteins, that is, they are expressed early in infection and in the absence of structural protein.

In a conference presentation (C. David Pauza, Immunization with Tat toxoid attenuates SHIV89.6PD infection in rhesus macaques, 12th Cent Gardes meeting, Mames-La-Coquette, 26.10.1999), experiments were described in which rhesus macaques were immunised with Tat toxoid alone or in combination with an envelope glycoprotein gp160 vaccine combination (one dose recombinant vaccinia virus and one dose recombinant protein). However, the results observed showed that the presence of the envelope glycoprotein gave no advantage over experiments performed with Tat alone.

However, we have found that a Tat- and/or Nef-containing immunogen (especially a Nef-Tat fusion protein) acts synergistically with gp120 in protecting rhesus monkeys from a pathogenic challenge with chimeric human-simian immunodeficiency virus (SHIV). To date the SHIV infection of rhesus macaques is considered to be the most relevant animal model for human AIDS. Therefore, we have used this preclinical model to evaluate the protective efficacy of vaccines containing a gp 120 antigen and a Nef- and Tat-containing antigen either alone or in combination. Analysis of two markers of viral infection and pathogenicity, the percentage of CD4-positive cells in the peripheral blood and the concentration of free SHIV RNA genomes in the plasma of the monkeys, indicated that the two antigens acted in synergy. Immunization with either gp 120 or NefTat + SIV Nef alone did not result in any difference compared to immunization with an adjuvant alone. In contrast, the administration of the combination of gp120 and NefTat + SIV Nef, antigens resulted in a marked improvement of the two above-mentioned parameters in all animals of those particular experimental group.

As described above, the NefTat protein, the SIV Nef protein and gp120 protein together give an enhanced response over that which is observed when either NefTat + SIV Nef, or gp120 are used alone. This enhanced response, or synergy can be seen in a decrease in viral load as a result of vaccination with these combined proteins. Alternatively, or additionally the enhanced response manifests itself by a maintenance of CD4+ levels over those levels found in the absence of vaccination with HIV NefTat, SIV Nef and HIV gp120. The synergistic effect is attributed to the combination of gp120 and Tat, or gp120 and Nef, or gp120 and both Nef and Tat.

It has been found that not only the Nef, Tat or NefTat proteins are advantageously combined or administered with the gp 120 protein. The same advantages are seen when DNA encoding Nef, Tat or NefTat is administered with gp 120 (protein or corresponding DNA).

It has been found that the proteins above, or DNA encoding the proteins, may advantageously be administered via a prime-boost strategy. In one aspect the present invention relates to such administration via a bombardment approach. Accordingly the invention provides the use of
a) an HIV Tat protein or polynucleotide; or
b) an HIV Nef protein or polynucleotide; or
c) an HIV Tat protein or polynucleotide linked to an HIV Nef protein or polynucleotide;
and an HIV gp120 protein or polynucleotide in the manufacture of a vaccine suitable for a prime-boost delivery for the prophylactic or therapeutic immunisation of humans against HIV, wherein the priming dose is the HIV gp 120 protein and HIV Tat protein and/or HIV Nef protein and the boosting dose is HIV gp 120 polynucleotide and HIV Tat polynucleotide and/or HIV Nef polynucleotide, and wherein the polynucleotide is delivered via a bombardment approach.

Numerous methods of carrying out a particle bombardment approach are known. See for example WO 91/07487. In one illustrative example, gas-driven particle acceleration can be achieved with devices such as those manufactured by Powderject Phanmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This offers a needle-free delivery approach wherein a dry powder formulation of microscopic particles, coated with a substance such as polynucleotide, is accelerated to high speed within a helium gas jet generated by a hand held device, propelling the particles into a target tissue of interest, typically the skin. The particles are preferably gold beads of a 0.4 - 4.0 µm, more preferably 0.6 - 2.0 µm diameter and the polynucleotide, preferably DNA is coated onto these and then encased in a cartridge for placing into the "gene gun".

The addition of other HIV proteins or DNA encoding them may further enhance the synergistic effect, which was observed between gp120 and Tat and/or Nef. These other proteins may also act synergistically with individual components of the gp120, Tat and/or Nef-containing vaccine, not requiring the presence of the full original antigen combination. The additional proteins may be regulatory proteins of HIV such as Rev, Vif, Vpu, and Vpr. They may also be structural proteins derived from the HIV *gag or pol* genes.

The HIV *gag* gene encodes a precursor protein p55, which can assemble spontaneously into immature virus-like particles (VLPs). The precursor is then proteolytically cleaved into the major structural proteins p24 (capsid) and p18 (matrix), and into several smaller proteins. Both the precursor protein p55 and its major derivatives p24 and p18 may be considered as appropriate vaccine antigens which may further enhance the synergistic effect observed between gp120 and Tat and/or Nef. The precursor p55 and the capsid protein p24 may be used as VLPs or as monomeric proteins.

The HIV Tat protein for use in the present invention may, optionally, be linked to an HIV Nef protein, for example as a fusion protein.

The HIV Tat protein, the HIV Nef protein or the NefTat fusion protein for use in the present invention may have a C terminal Histidine tail which preferably comprises Between 5-10 Histidine residues. The presence of an histidine (or 'His') tail aids purification.

In a preferred embodiment the proteins are expressed with a Histidine tail comprising between 5 to 10 and preferably six Histidine residues. These are advantageous in aiding purification. Separate expression, in yeast (Saccharomyces cerevisiae), of Nef (Macreadie I.G. et al., 1993, Yeast 9 (6) 565-573) and Tat (Braddock M et al., 1989, Cell 58 (2) 269-79) has been reported. Nef protein and the Gag proteins p55 and p18 arc myristoylated. The expression of Nef and Tat separately in a Pichia expression system (Nef-His and Tat-His constructs), and the expression of a fusion construct Nef-Tat-His have been described previously in WO99/16884.

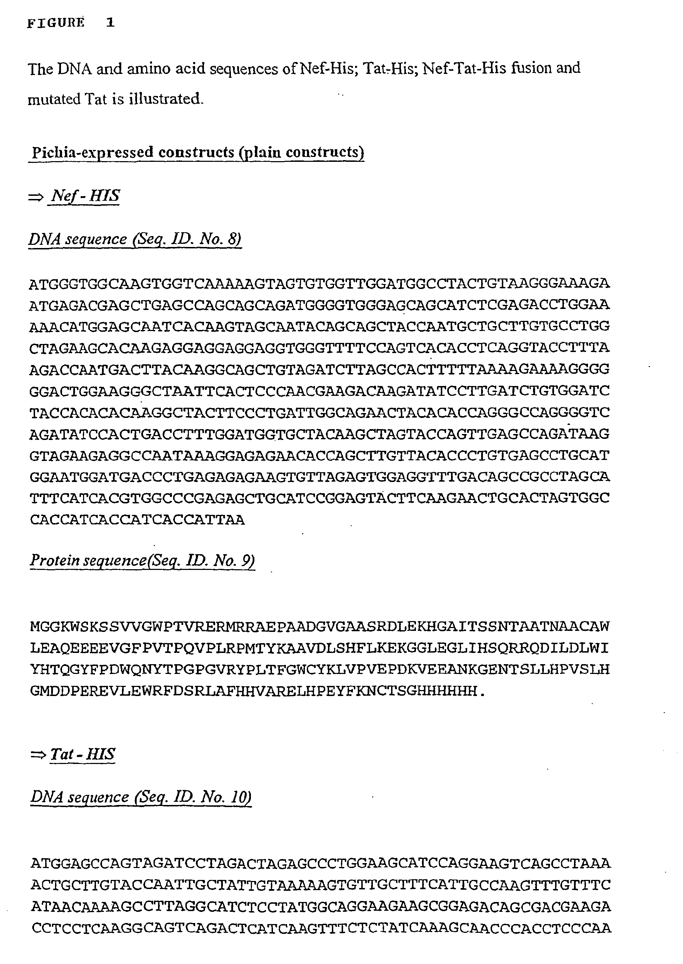

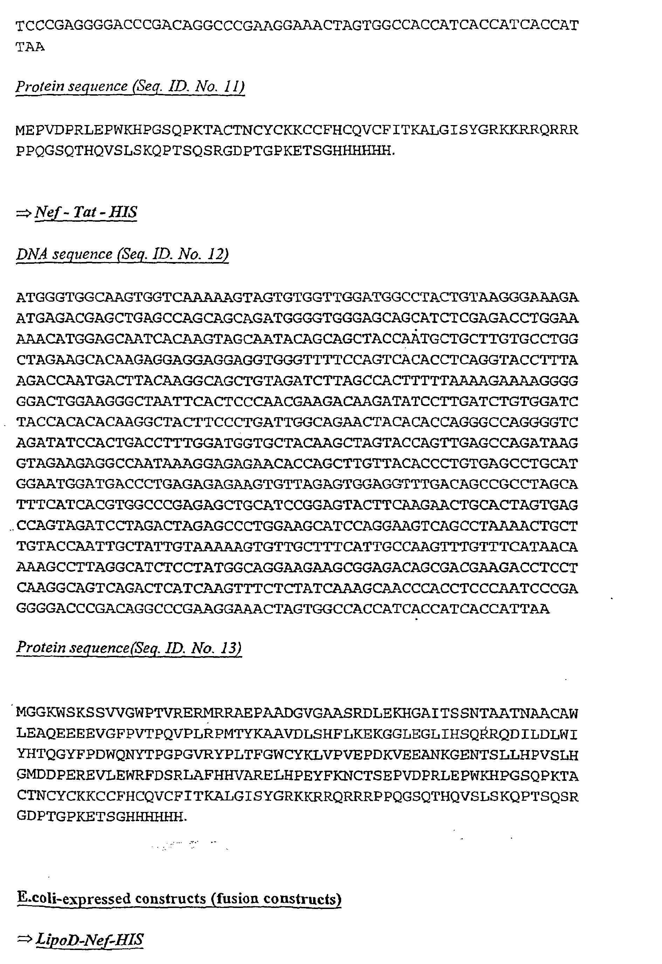

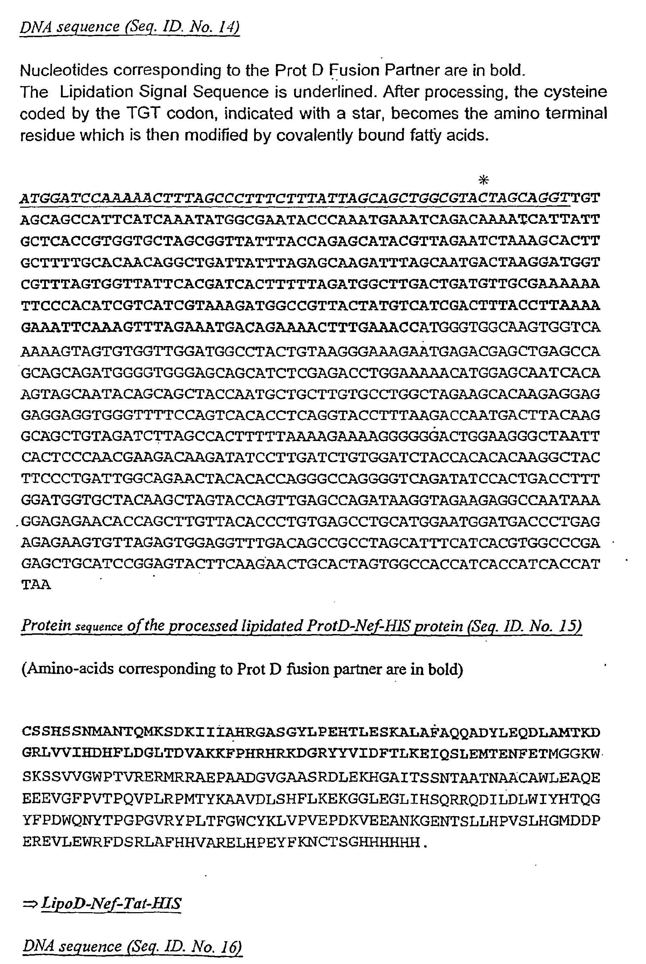

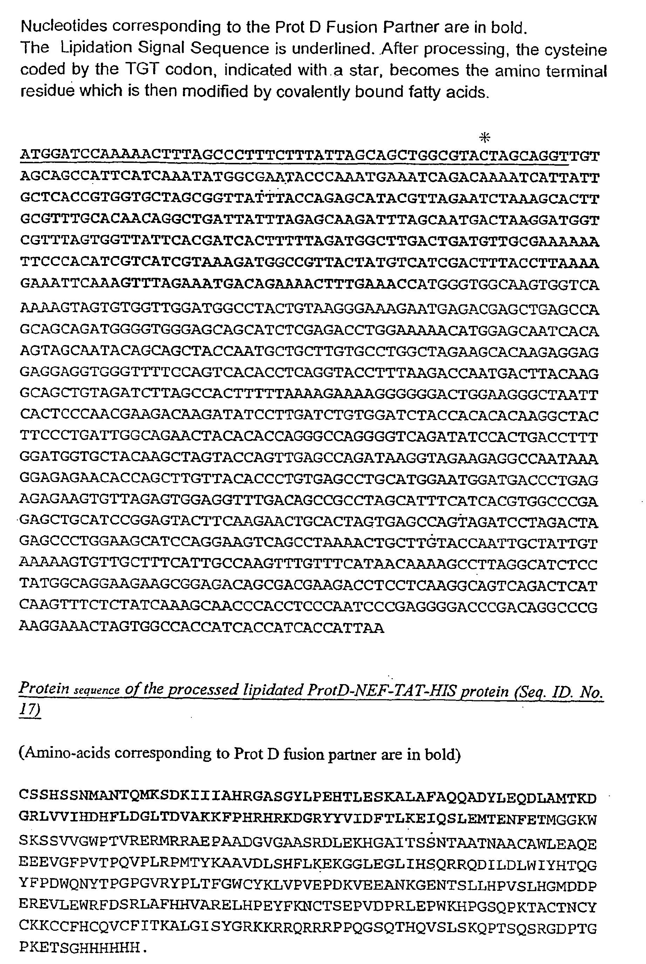

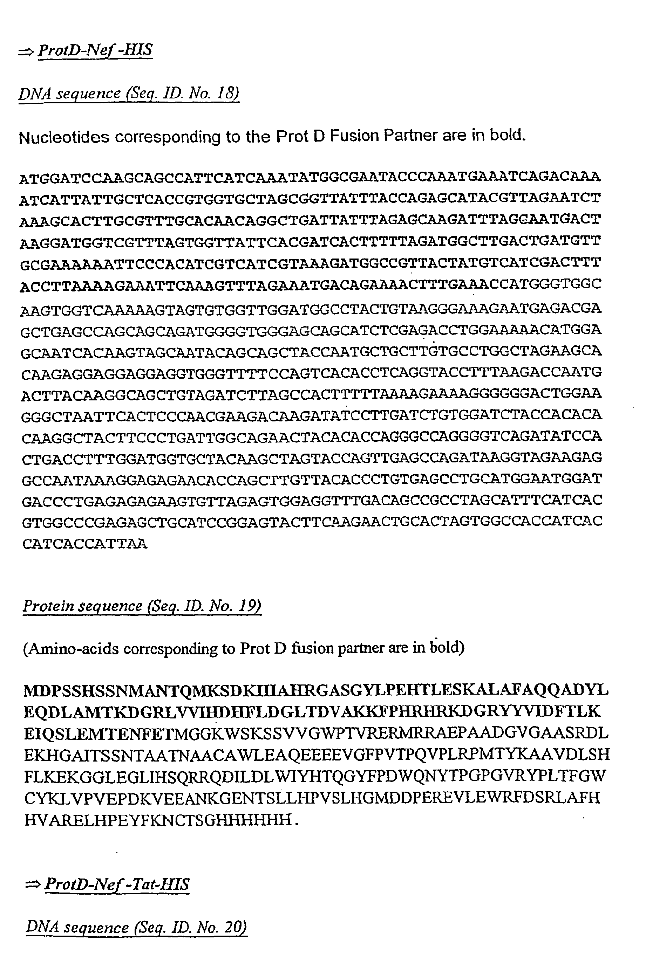

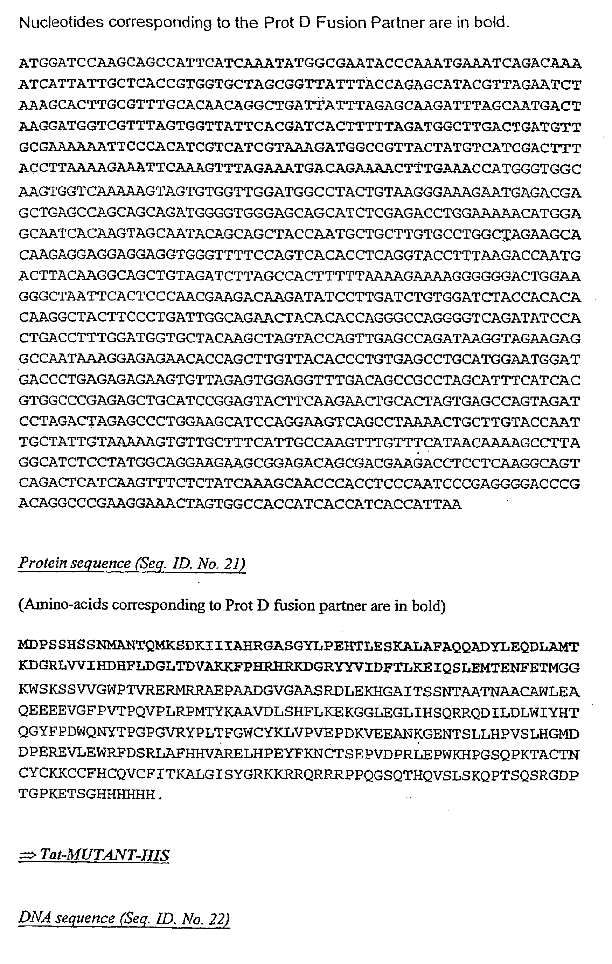

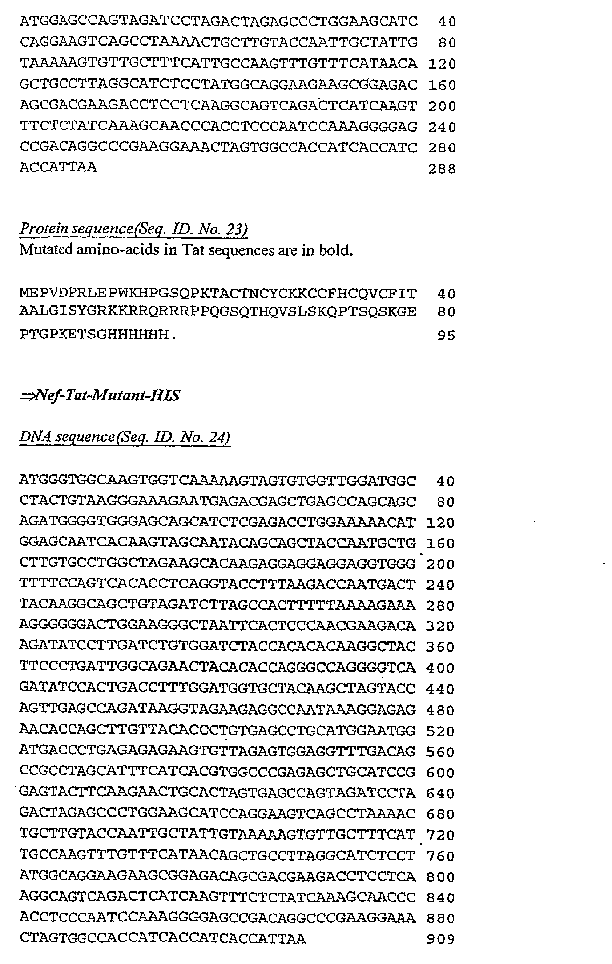

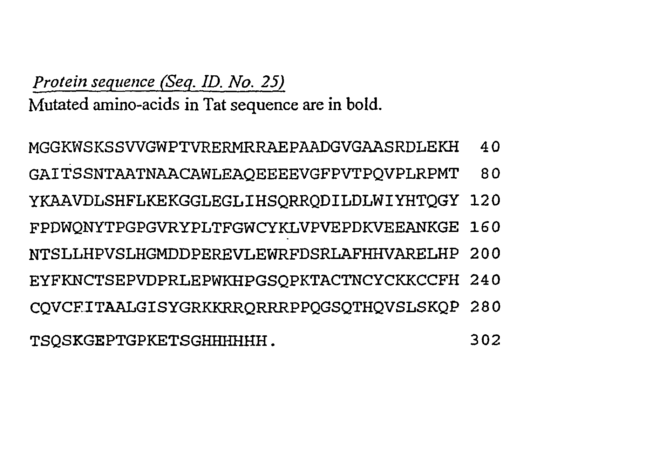

The DNA and amino acid sequences of representative Nef-His (Seq. ID. No.s 8 and 9), Tat-His (Scq. ID. No.s 10 and 11) and of Nef-Tat-His fusion proteins (Seq. ID. No.s 12 and 13) are set forth in Figure 1.

The HIV proteins may be used in their native conformation, or more preferably, may be modified for vaccine use. These modifications may either be required for technical reasons relating to the method of purification, or they may be used to biologically inactivate one or several functional properties of the Tat or Nef protein. Thus the invention encompasses the use of derivatives of HIV proteins or polynucleotides, particularly DNAs, which may be, for example, mutated. The term 'mutated' is used herein to mean a DNA or protein molecule which has undergone deletion, addition or substitution of one or more nucleotides or amino acids using well known techniques for site directed mutagenesis or any other conventional method.

For example, a mutant Tat protein may be mutated so that it is biologically inactive whilst still maintaining its immunogenic epitopes. One possible mutated tat gene, constructed by D.Clements (Tulane University), (originating from BH10 molecular clone) bears mutations in the active site region (Lys41→Ala)and in RGD motif (Arg78→Lys and Asp80→Glu) (Virology 235: 48-64, 1997).

A mutated Tat is illustrated in Figure 1 (Seq. ID. No.s 22 and 23) as is a Nef-Tat Mutant-His (Seq. ID. No.s 24 and 25).

The HIV Tat or Nef proteins for use in the present invention may be modified by chemical methods during the purification process to render the proteins stable and monomeric. One method to prevent oxidative aggregation of a protein such as Tat or Nef is the use of chemical modifications of the protein's thiol groups. In a first step the disulphide bridges are reduced by treatment with a reducing agent such as DTT, beta-mercaptoethanol, or gluthatione. In a second step the resulting thiols are blocked by reaction with an alkylating agent (for example, the protein can be caused to react with iodoacetamide). Such chemical modification does not modify functional properties of Tat or Nef as assessed by cell binding assays and inhibition of lymphoproliferation of human peripheral blood mononuclear cells.

It will be understood that the invention also emcompasses the use of fragments of the full length proteins provided that the fragments comprise at least one immunogenic epitope.

The HIV Tat protein and HIV gp120 proteins can be purified by the methods outlined in the attached examples.

An immunoprotective or immunotherapeutic quantity of the Tat and/or Nef or NefTat and gp120 components (protein or DNA) for use in the invention may be prepared by conventional techniques.

Vaccine preparation is generally described in New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

The amount of protein in a vaccine dose is selected as an amount which induces an immunoprotective response without significant, adverse side effects in typical vaccinees. Such amount will vary depending upon which specific immunogen is employed. Generally, it is expected that each dose will comprise 1-1000 µg of each protein, preferably 2-200 µg, most preferably 4-40 µg of Tat or Nef or NefTat and preferably 1-150 µg, most preferably 2-25 µg of gp120. An optimal amount for a particular vaccine can be ascertained by standard studies involving observation of antibody titres and other responses in subjects. One particular example of a vaccine dose will comprise 20 µg of NefTat and 5 or 20 µg of gp120. Following an initial vaccination, subjects may receive a boost in about 4 weeks, and a subsequent second booster immunisation.

The proteins of the present invention are preferably adjuvanted in a vaccine formulation of the invention. The polynucleotides used in the present invention are optionally adjuvanted, and may be delivered in a formulation with an adjuvant or separately from the adjuvant, either simultaneously or sequentially. Adjuvants are described in general in Vaccine Design - the Subunit and Adjuvant Approach, edited by Powell and Newman, Plenum Press, New York, 1995.

Suitable adjuvants include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, or polyphosphazenes.

In the formulation of vaccines for use in the invention it is preferred that the adjuvant composition induces a preferential Th1 response. However it will be understood that other responses, including other humoral responses, are not excluded.

An immune response is generated to an antigen through the interaction of the antigen with the cells of the immune system. The resultant immune response may be broadly distinguished into two extreme catagories, being humoral or cell mediated immune responses (traditionally characterised by antibody and cellular effector mechanisms of protection respectively). These categories of response have been termed Th1-type responses (cell-mediated response), and Th2-type immune responses (humoral response).

Extreme Th1-type immune responses may be characterised by the generation of antigen specific, haplotype restricted cytotoxic T lymphocytes, and natural killer cell responses. In mice Th1-type responses are often characterised by the generation of antibodies of the IgG2a subtype, whilst in the human these correspond to IgG1 type antibodies. Th2-type immune responses are characterised by the generation of a broad range of immunoglobulin isotypes including in mice IgG1, IgA, and IgM.

It can be considered that the driving force behind the development of these two types of immune responses are cytokines, a number of identified protein messengers which serve to help the cells of the immune system and steer the eventual immune response to either a Th1 or Th2 response. Thus high levels of Th1-type cytokines tend to favour the induction of cell mediated immune responses to the given antigen, whilst high levels of Th2-type cytokines tend to favour the induction of humoral immune responses to the antigen.

It is important to remember that the distinction of Th1 and Th2-type immune responses is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman *(*Mosmann, T.R. and Coffman, R.L. (1989) TH1 and TH2 cells: different patterns of lymphokine secretion lead to different functional properties. Annual Review of Immunology, 7, p145-173*).* Traditionally, Th1-type responses are associated with the production of the INF-γ and IL-2 cytokines by T-lymphocytes. Other cytokines often directly associated with the induction of Th1-type immune responses are not produced by T-cells, such as 1L-12. In contrast, Th2- type responses are associated with the secretion of IL-4, IL-5, IL-6, IL-10 and tumour necrosis factor-β(TNF-β).

It is known that certain vaccine adjuvants are particularly suited to the stimulation of either Th1 or Th2 - type cytokines responses. Traditionally the best indicators of the Th1:Th2 balance of the immune response after a vaccination or infection includes direct measurement of the production of Th1 or Th2 cytokines by T lymphocytes *in vitro* after restimulation with antigen, and/or the measurement of the IgG1:IgG2a ratio of antigen specific antibody responses.

Thus, a Th1-type adjuvant is one which stimulates isolated T-cell populations to produce high levels of Th1-type cytokines when re-stimulated with antigen *in vitro,* and induces antigen specific imnunoglobulin responses associated with Th1-type isotype.

Preferred Th1-type immunostimulants which may be formulated to produce adjuvants suitable for use in the present invention include and are not restricted to the following.

Monophosphoryl lipid A, in particular 3-de-O-acylated monophosphoryl lipid A (3D-MPL), is a preferred Th1-type immunostimulant for use in the invention. 3D-MPL is a well known adjuvant manufactured by Ribi Immunochem, Montana. Chemically it is often supplied as a mixture of 3-de-O-acylated monophosphoryl lipid A with either 4, 5, or 6 acylated chains. It can be purified and prepared by the methods taught in GB 2122204B, which reference also discloses the preparation of diphosphoryl lipid A, and 3-O-deacylated variants thereof. Other purified and synthetic lipopolysaccharides have been described (US 6,005,099 and EP 0 729 473 B1; Hilgers et al., 1986, Int.Anch.Allergy.Immunol., 79(4):392-6; Hilgers et al., 1987, Immunology, 60(1):141-6; and EP 0 549 074 B1). A preferred form of 3D-MPL is in the form of a particulate formulation having a small particle size less than 0.2µm in diameter, and its method of manufacture is disclosed in EP 0 689 454.

Saponins are also preferred Th1 immunostimulants in accordance with the invention. Saponins are well known adjuvants and are taught in: Lacaille-Dubois, M and Wagner H. (1996. A review of the biological and pharmacological activities of saponins. Phytomedicine vol 2 pp 363-386). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1. The haemolytic saponins QS21 and QS17 (HPLC purified fractions of Quil A) have been described as potent systemic adjuvants, and the method of their production is disclosed in US Patent No. 5,057,540 and EP 0 362 279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Another preferred immunostimulant is an immunostimulatory oligonucleotide containing unmethylated CpG dinucleotides ("CpG"). CpG is an abbreviation for cytosine-guanosine dinucleotide motifs present in DNA. CpG is known in the art as being an adjuvant when administered by both systemic and mucosal routes (WO 96/02555, EP 468520, Davis et al., J.Immunol, 1998, 160(2):870-876; McCluskie and Davis, J.Immunol., 1998, 161(9):4463-6). Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the CG motif is not methylated, but other unmethylated CpG sequences are known to be immunostimulatory and may be used in the present invention.

In certain combinations of the six nucleotides a palindromic sequence is present. Several of these motifs, either as repeats of one motif or a combination of different motifs, can be present in the same oligonucleotide. The presence of one or more of these immunostimulatory sequences containing oligonucleotides can activate various immune subsets, including natural killer cells (which produce interferon γ and have cytolytic activity) and macrophages (Wooldrige et al Vol 89 (no. 8), 1977). Other unmethylated CpG containing sequences not having this consensus sequence have also now been shown to be immunomodulatory.

CpG when formulated into vaccines, is generally administered in free solution together with free antigen (WO 96/02555; McCluskie and Davis, *supra)* or covalently conjugated to an antigen (WO 98/16247), or formulated with a carrier such as aluminium hydroxide ((Hepatitis surface antigen) Davis *et al. supra ;* Brazolot-Millan et al., Proc.Natl.Acad.Sci., USA, 1998, 95(26), 15553-8).

Such immunostimulants as described above may be formulated together with carriers, such as for example liposomes, oil in water emulsions, and or metallic salts, including aluminium salts (such as aluminium hydroxide). For example, 3D-MPL may be formulated with aluminium hydroxide (EP 0 689 454) or oil in water emulsions (WO 95/17210); QS21 may be advantageously formulated with cholesterol containing liposomes (WO 96/33739), oil in water emulsions (WO 95/17210) or alum (WO 98/15287); CpG may be formulated with alum (Davis *et al. supra;* Brazolot-Millan *supra)* or with other cationic carriers.

Combinations of immunostimulants are also preferred, in particular a combination of a monophosphoryl lipid A and a saponin derivative (WO 94/00153; WO 95/17210; WO 96/33739; WO 98/56414; WO 99/12565; WO 99/11241), more particularly the combination of QS21 and 3D-MPL as disclosed in WO 94/00153. Alternatively, a combination of CpG plus a saponin such as QS21 also forms a potent adjuvant for use in the present invention.

Thus, suitable adjuvant systems include, for example, a combination of monophosphoryl lipid A, preferably 3D-MPL, together with an aluminium salt. An enhanced system involves the combination of a monophosphoryl lipid A and a saponin derivative particularly the combination of QS21 and 3D-MPL as disclosed in WO 94/00153, or a less reactogenic composition where the QS21 is quenched in cholesterol containing liposomes (DQ) as disclosed in WO 96/33739.

A particularly potent adjuvant formulation involving QS21, 3D-MPL & tocopherol in an oil in water emulsion is described in WO 95/17210 and is another preferred formulation for use in the invention.

Another preferred formulation comprises a CpG oligonucleotide alone or together with an aluminium salt.

Particularly preferred adjuvant and/or carrier combinations are as follows:
i) 3D-MPL + QS21 in DQ
ii) Alum + 3D-MPL
iii) Alum + QS21 in DQ + 3D-MPL
iv) Alum + CpG
v) 3D-MPL + QS21 in DQ + oil in water emulsion
vi) CpG

As already noted, the vaccine may contain polynucleotide, preferably DNA, encoding one or more of the Tat, Nef and gp120 polypeptides, such that the polypeptide is generated *in situ.*

The DNA constructs *per se,* especially those described herein, also form part of the invention.

The polynucleotide may be present within any of a variety of delivery systems known to those of ordinary skill in the art, including nucleic acid expression systems such as plasmid DNA, bacteria and viral expression systems. Numerous gene delivery techniques are well known in the art, such as those described by Rolland, Crit. Rev. Therap. Drug Carrier Systems 15:143-198, 1998 and references cited therein. Plasmid based delivery of genes, particularly for immunisation or gene therapy purposes is known. For example, administration of naked DNA by injection into mouse muscle is outlined by Vical in International Patent Application WO90/11092.

Johnston et al WO 91/07457 describe methods of transferring a gene to vertebrate cells, by the use of microprojectiles that have been coated with a polynucleotide encoding a gene of interest, and accelerating the microparticles such that the microparticles can penetrate the target cell.

DNA vaccines usually consist of a bacterial plasmid vector into which is inserted a strong viral promoter, the gene of interest which encodes for an antigenic peptide and a polyadenylation/transcriptional termination sequences. The gene of interest may encode a full protein, a fusion protein comprising different antigens, or simply an antigenic peptide sequence relating to the pathogen, tumour or other agent which is intended to be protected against. Thus the plasmid may encode a fragment of a full protein, provided that the fragment comprises at least one immunogenic epitope of the full protein. The plasmid can be grown in bacteria, such as for example E.coli and then isolated and prepared in an appropriate medium, depending upon the intended route of administration, before being administered to the host. Following administration the plasmid is taken up by cells of the host where the encoded peptide is produced. The plasmid vector will preferably be made without an origin of replication which is functional in eukaryotic cells, in order to prevent plasmid replication in the mammalian host and integration within chromosomal DNA of the animal concerned. All of these features may apply singly or in combination to the present invention.

There are a number of advantages of DNA vaccination relative to traditional vaccination techniques. First, it is predicted that because the proteins which are encoded by the DNA sequence are synthesised in the host, the structure or conformation of the protein will be similar to the native protein associated with the disease state. It is also likely that DNA vaccination will offer protection against different strains of a virus, by generating a cytotoxic T lymphocyte response that recognises epitopes from conserved proteins. Furthermore, because the plasmids are taken up by the host cells where antigenic protein can be produced, a long-lasting immune response will be elicited. The technology also offers the possibility of combining diverse immunogens into a single preparation to facilitate simultaneous immunisation in relation to a number of disease states.

Helpful background information in relation to DNA vaccination is provided in Donnelly et al "DNA vaccines" Ann. Rev Immunol. 1997 15: 617-648.

In one preferred embodiment the DNA can be delivered via a particle bombardment approach e.g. a "gene gun" approach as described hereinabove.

Appropriate nucleic acid expression systems contain the necessary DNA sequences for expression in the patient (such as a suitable promoter and terminating signal). When the expression system is a recombinant live microorganism, such as a virus or bacterium, the gene of interest can be inserted into the genome of a live recombinant virus or bacterium. Inoculation and *in vivo* infection with this live vector will lead to *in vivo* expression of the antigen and induction of immune responses. Viruses and bacteria used for this purpose are for instance: poxviruses (e.g; vaccinia, fowlpox, canarypox, modified poxviruses e.g. Modified Virus Ankara (MVA)), alphaviruses (Sindbis virus, Semliki Forest Virus, Venezuelian Equine Encephalitis Virus), flaviviruses (yellow fever virus, Dengue virus, Japanese encephalitis virus), adenoviruses, adeno-associated virus, picomaviruses (poliovirus, rhinovirus), herpesviruses (varicella zoster virus, etc), Listeria, Salmonella , Shigella, Neisseria, BCG. These viruses and bacteria can be virulent, or attenuated in various ways in order to obtain live vaccines.

Thus, the Nef, Tat and gp 120 components of a preferred vaccine according to the invention may be provided in the form of polynucleotides or recombinant DNA encoding the desired proteins. The polynucleotides employed in the invention may encode a full protein, a fusion protein comprising different antigens, or one or more antigenic peptide sequences. Thus the polynucleotides may encode a fragment of a full protein, provided that the fragment comprises at least one immunogenic epitope of the full protein.

At least one of the DNAs for Nef, Tat, NefTat or gp 120 may preferably be codon optimised as described, for example, in Andre S. Seed B. Eberle J. Schraut W. Bultmann A. Haas J. (1998): Increased immune response elicited by DNA vaccination with a synthetic gp120 sequence with optimized codon usage, Journal of Virology. 72(2):1497-503. In one preferred aspect the DNA encoding gp120 is codon optimised.

Codon optimisation is used to optimise the polynucleotide sequences for expression in mammalian cells. That is the sequence is optimised to resemble the codon usage of genes in mammalian cells.

In one embodiment of the present invention the Nef, Tat, NefTat or gp120 polynucleotide sequence has a codon usage pattern which resembles that of highly expressed mammalian genes, particularly human genes. Preferably the polynucleotide sequence is a DNA sequence. Desirably the codon usage pattern of the polynucleotide sequence is typical of highly expressed human genes.

The DNA code has 4 letters (A, T, C and G) and uses these to spell three letter "codons" which represent the amino acids the proteins encodes in an organism's genes. The linear sequence of codons along the DNA molecule is translated into the linear sequence of amino acids in the protein(s) encoded by those genes. The code is highly degenerate, with 61 codons coding for the 20 natural amino acids and 3 codons representing "stop" signals. Thus, most amino acids are coded for by more than one codon - in fact several are coded for by four or more different codons.

Where more than one codon is available to code for a given amino acid, it has been observed that the codon usage patterns of organisms are highly non-random. Different species show a different bias in their codon selection and, furthermore, utilisation of codons may be markedly different in a single species between genes which are expressed at high and low levels. This bias is different in viruses, plants, bacteria and mammalian cells, and some species show a stronger bias away from a random codon selection than others. For example, humans and other mammals are less strongly biased than certain bacteria or viruses. For these reasons, there is a significant probability that a mammalian gene expressed in E.coli or a viral gene expressed in mammalian cells will have an inappropriate distribution of codons for efficient expression. It is believed that the presence in a heterologous DNA sequence of clusters of codons which are rarely observed in the host in which expression is to occur, is predictive of low heterologous expression levels in that host.

In the polynucleotides of the present invention, the codon usage pattern is altered from that typical of human immunodeficiency viruses to more closely represent the codon bias of the target organism, e.g. a mammal, especially a human. The "codon usage coefficient" is a measure of how closely the codon pattern of a given polynucleotide sequence resembles that of a target species. Codon frequencies can be derived from literature sources for the highly expressed genes of many species (see e.g. Nakamura et.al. Nucleic Acids Research 1996, 24:214-215). The codon frequencies for each of the 61 codons (expressed as the number of occurrences occurrence per 1000 codons of the selected class of genes) are normalised for each of the twenty natural amino acids, so that the value for the most frequently used codon for each amino acid is set to 1 and the frequencies for the less common codons are scaled to lie between zero and 1. Thus each of the 61 codons is assigned a value of 1 or lower for the highly expressed genes of the target species. In order to calculate a codon usage coefficient for a specific polynucleotide, relative to the highly expressed genes of that species, the scaled value for each codon of the specific polynucleotide are noted and the geometric mean of all these values is taken (by dividing the sum of the natural logs of these values by the total number of codons and take the anti-log). The coefficient will have a value between zero and 1 and the higher the coefficient the more codons in the polynucleotide are frequently used codons. If a polynucleotide sequence has a codon usage coefficient of 1, all of the codons are "most frequent" codons for highly expressed genes of the target species.

According to the present invention, the codon usage pattern of the polynucleotide will preferably exclude rare codons representing less than 10% of the codon use for an amino acid in highly expressed genes of the target organism. In an alternative preferred embodiment, the polynucleotide will exclude codons with an RSCU value of less than 0.2 in highly expressed genes of the target organism. A relative synonymous codon usage (RSCU) value is the observed number of codons divided by the number expected if all codons for that amino acid were used equally frequently. A polynucleotide of the present invention will generally have a codon usage coefficient for highly expressed human genes of greater than 0.3, preferably greater than 0.4, most preferably greater than 0.5. Codon usage tables for human can also be found in Genebank.

In comparison, a highly expressed beta action gene has a RSCU of 0.747. The codon usage table for a *homo sapiens* is set out below:
Codon Usage Table:
***Homo sapiens* [gbpri]: 27143 CDS's (12816923 codons) Standard Codon Usage Table**
fields: [triplet] [frequency: **per thousand**] ([number])

| | | | | | | | |
|---|---|---|---|---|---|---|---|
| UUU | 17.0(217684) | UCU | 14.8(189419) | UAU | 12.1(155645) | UGU | 10.0(127719) |
| UUC | 20.5(262753) | UCC | 17.5(224470) | UAC | 15.8(202481) | UGC | 12.3(157257) |
| UUA | 7.3( 93924) | UCA | 11.9(152074) | UAA | 0.7( 9195) | UGA | 1.3( 16025) |
| UUG | 12.5(159611) | UCG | 4.5( 57572) | UAG | 0.5( 6789) | UGG | 12.9(165930) |
| CUU | 12.8(163707) | CCU | 17.3(222146) | CAU | 10.5(134186) | CGU | 4.6( 59454) |
| CUC | 19.3(247391) | CCC | 20.0(256235) | CAC | 14.9(190928) | CGC | 10.8(137865) |
| CUA | 7.0( 89078) | CCA | 16.7(214583) | CAA | 12.0(153590) | CGA | 6.3( 80709) |
| CUG | 39.7(509096) | CCG | 7.0( 89619) | CAG | 34.5(441727) | CGG | 11.6(148666) |
| AUU | 15.8(202844) | ACU | 12.9(165392) | AAU | 17.0(218508) | AGU | 12.0(154442) |
| AUC | 21.6(277066) | ACC | 19.3(247805) | AAC | 19.8(253475) | AGC | 19.3(247583) |
| AUA | 7.2( 92133) | ACA | 14.9(191518) | AAA | 24.0(308123) | AGA | 11.5(147264) |
| AUG | 22.3(285776) | ACG | 6.3 ( 80369) | AAG | 32.6(418141) | AGG | 11.3(145276) |
| GUU | 10.9(139611) | GCU | 18.5(236639) | GAU | 22.4(286742) | GGU | 10.8(138606) |
| GUC | 14.6(187333) | GCC | 28.3(362086) | GAC | 26.1(334158) | GGC | 22.7(290904) |
| GUA | 7.0( 89644) | GCA | 15.9(203310) | GAA | 29.1(373151) | GGA | 16.4(210643) |
| GUG | 28.8(369006) | GCG | 7.5( 96455) | GAG | 40.2(515485) | GGG | 16.4(209907) |

Coding GC 52.51% 1st letter GC 56.04% 2nd letter GC 42.35% 3rd letter GC 59.13%.

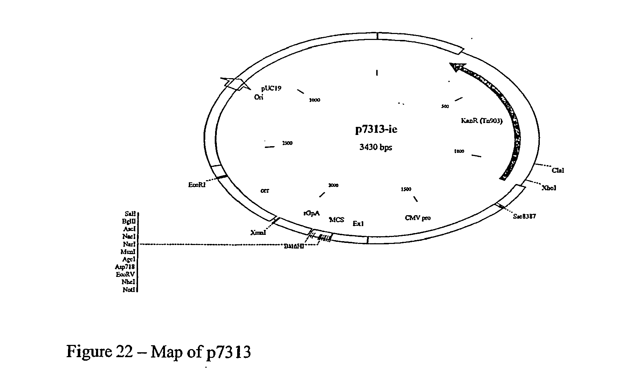

According to a further aspect of the invention, an expression vector is provided which comprises and is capable of directing the expression of a Nef and/or Tat or NefTat, and a gp120 polynucleotide sequence according to the first aspect of the invention, in particular where the codon usage pattern of at least one of the Nef, Tat, NefTat or gp120 polynucleotide sequences, particularly the gp120 sequence, is typical of highly expressed mammalian genes, preferably highly expressed human genes. The vector is suitable for driving expression of heterologous DNA in mammalian cells, particularly human cells. In one embodiment, the expression vector is p7313 (see figure 22).

In a further aspect the invention provides a plurality of particles, preferably gold particles, coated with DNA comprising one or more vectors encoding gp120 and nef and/or tat or neftat. Preferably the particles are coated with a single vector which encodes gp120 and nef and tat, the latter most preferably in the form of a NefTat fusion protein. Most preferably one or more of the sequences are codon optimised for expression in human cells.

In a preferred aspect the DNA encoding the nef, tat and gp 120 is present on a single vector.

Preferably the vector comprises the nef, tat and gp 120 sequences inserted 3' to an enhanced HCMV IE1 promoter for efficient expression. This is preferably the HCMV immediate early promoter devoid of intron A, but including exon 1.

One suitable vector according to the invention is that denoted as p7313, further described below.

The vectors which comprise the nucleotide sequences described herein are administered in such amount as will be prophylactically or therapeutically effective. The quantity to be administered is generally in the range of one picogram to 1 milligram, preferably 1 picogram to 10 micrograms for particle-mediated delivery as described herein. The exact quantity may vary depending on the weight of the patient being immunised and the precise route of administration.

Immunisations according to the invention may be performed with a combination of protein and DNA-based formulations. Adjuvanted protein vaccines induce mainly antibodies and T helper immune responses, while delivery of DNA as a plasmid or a live vector induces strong cytotoxic T lymphocyte (CTL) responses. Thus, the combination of protein and DNA vaccination will provide for a wide variety of immune responses. This is particularly relevant in the context of HIV, since both neutralising antibodies and CTL are thought to be important for the immune defence against HIV.

The DNA may be delivered as plasmid DNA or in the form of a recombinant live vector, e.g. a poxvirus vector or any other suitable live vector such as those described herein. Protein antigens may be injected once or several times followed by one or more DNA administrations, or DNA may be used first for one or more administrations followed by one or more protein immunisations.

A particular example of prime-boost immunisation according to the invention involves priming with DNA in the form of a recombinant live vector such as a modified poxvirus vector, for example Modified Virus Ankara (MVA) or a derivative thereof e.g through passaging or genetic manipulation, or an alphavirus vector for example Venezuelian Equine Encephalitis Virus, followed by boosting with a protein, preferably an adjuvanted protein. Optionally the DNA is adjuvanted with a suitable DNA vaccine adjuvant known in the art.

The invention is illustrated in the accompanying examples and Figures:

### EXAMPLES

### General

The Nef gene from the Bru/Lai isolate (Cell 40: 9-17, 1985) was selected for the constructs of these experiments since this gene is among those that are most closely related to the consensus Nef.

The starting material for the Bru/Lai Nef gene was a 1170bp DNA fragment cloned on the mammalian expression vector pcDNA3 (pcDNA3/Nef).

The Tat gene originates from the BH10 molecular clone. This gene was received as an HTLV III cDNA clone named pCV1 and described in Science, 229, p69-73, 1985.

The expression of the Nef and Tat genes could be in Pichia or any other host.

### Example 1. EXPRESSION OF HIV-1 nef AND tat SEQUENCES IN PICHIA PASTORIS.

Nef protein, Tat protein and the fusion Nef -Tat were expressed in the methylotrophic yeast *Pichia pastoris* under the control of the inducible alcohol oxidase (AOX1) promoter.

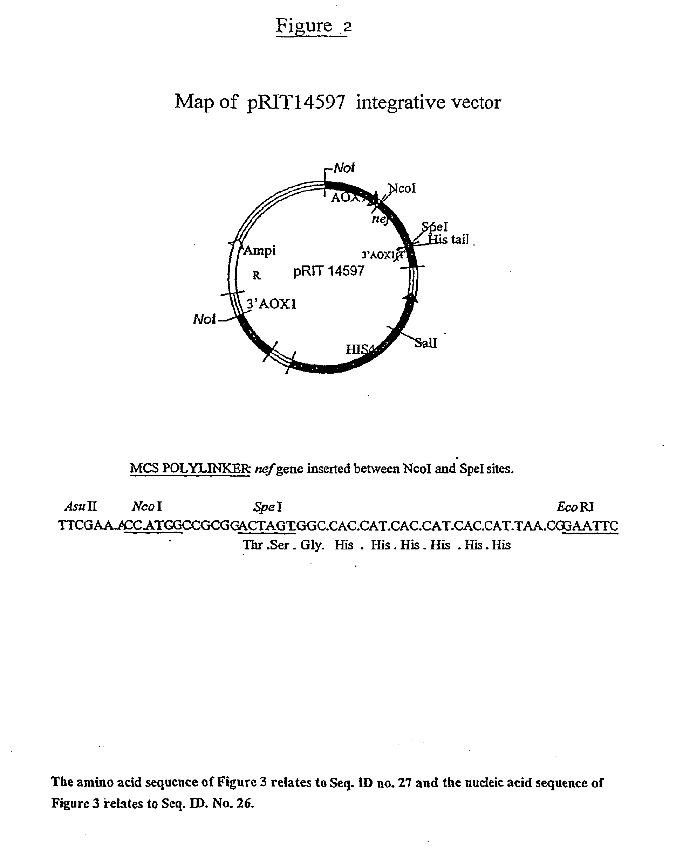

To express these HIV-1 genes a modified version of the integrative vector PHIL-D2 (INVITROGEN) was used. This vector was modified in such a way that expression of heterologous protein starts immediately after the native ATG codon of the AOX1 gene and will produce recombinant protein with a tail of one glycine and six histidines residues . This PHIL-D2-MOD vector was constructed by cloning an oligonucleotide linker between the adjacent AsuII and EcoRI sites of PHIL-D2 vector (see Figure 2 which includes Seq ID Nos: 26 and 27). In addition to the His tail, this linker carries NcoI, SpeI and XbaI restriction sites between which *nef, tat* and *nef-tat* fusion were inserted.

### 1.1 CONSTRUCTION OF THE INTEGRATIVE VECTORS pRIT14597 (encoding Nef-His protein), pRIT14598 (encoding Tat-His protein) and pRIT14599 (encoding fusion Nef-Tat-His).

The *nef gene* was amplified by PCR from the pcDNA3/Nef plasmid with primers 01 and 02.

NcoI
PRIMER 01 (Seq ID NO 1): 5'ATCGTCCATG.GGT.GGC.AAG.TGG.T 3'

SpeI
PRIMER 02 (Seq ID NO 2): 5' CGGCTACTAGTGCAGTTCTTGAA 3'

The PCR fragment obtained and the integrative PHIL-D2-MOD vector were both restricted by NcoI and SpeI, purified on agarose gel and ligated to create the integrative plasmid pRIT14597 (see Figure 2).

The *tat* gene was amplified by PCR from a derivative of the pCV1 plasmid with primers 05 and 04:

SpeI
PRIMER 04 (Seq ID NO 4): 5' CGGCTACTAGTTTCCTTCGGGCCT 3'

NcoI
PRIMER 05 (Seq ID NO 5): 5'ATCGTCCATGGAGCCAGTAGATC 3'

An NcoI restriction site was introduced at the 5' end of the PCR fragment while a SpeI site was introduced at the 3' end with primer 04. The PCR fragment obtained and the PHIL-D2-MOD vector were both restricted by NcoI and SpeIl, purified on agarose gel and ligated to create the integrative plasmid pRIT14598.

To construct pRIT14599, a 910bp DNA fragment corresponding to the *nef-tat*-His coding sequence was ligated between the EcoRI blunted(T4 polymerase) and NcoI sites of the PHIL-D2-MOD vector. The *nef-tat*-His coding fragment was obtained by XbaI blunted(T4 polymerase) and NcoI digestions of pRIT14596.

### 1.2 TRANSFORMATION OF PICHIA PASTORIS STRAIN GS115(his4).

To obtain *Pichia pastoris* strains expressing Nef-His, Tat-His and the fusion Nef-Tat-His, strain GS 115 was transformed with linear NotI fragments carrying the respective expression cassettes plus the HIS4 gene to complement his4 in the host genome.Transformation of GS115 with NotI-linear fragments favors recombination at the AOXI locus.

Multicopy integrant clones were selected by quantitative dot blot analysis and the type of integration, insertion (Mut⁺phenotype) or transplacement (Mut^{s}phenotype), was determined.

From each transformation, one transformant showing a high production level for the recombinant protein was selected :
Strain Y1738 (Mut⁺ phenotype) producing the recombinant Nef-His protein,
a myristylated 215 amino acids protein which is composed of:
   °Myristic acid
   °A methionine, created by the use of NcoI cloning site of PHIL-D2-MOD vector
   o205 a.a. ofNefprotein(starting at a.a.2 and extending to a.a.206)
   oA threonine and a serine created by the cloning procedure (cloning at SpeI site of PHIL-D2-MOD vector.
   oOne glycine and six histidines.
Strain Y1739 (Mut⁺ phenotype) producing the Tat-His protein, a 95 amino acid protein which is composed of:
   oA methionine created by the use of NcoI cloning site o85 a.a. of the Tat protein(starting at a.a.2 and extending to a.a.86)
   oA threonine and a serine introduced by cloning procedure oOne glycine and six histidines
Strain Y1737(Mut^{s} phenotype) producing the recombinant Nef-Tat-His fusion protein, a myristylated 302 amino acids protein which is composed of:
   °oyristic acid
   °A methionine, created by the use of NcoI cloning site °205a.a. of Nef protein(starting at a.a.2 and extending to a.a.206)
   °A threonine and a serine created by the cloning procedure °85a.a. of the Tat protein(starting at a.a.2 and extending to a.a.86)
   °A threonine and a serine introduced by the cloning procedure °One glycine and six histidines

### Example 2. EXPRESSION OF HIV-1 Tat-MUTANT IN PICHIA PASTORIS

A mutant recombinant Tat protein has also been expressed. The mutant Tat protein must be **biologically inactive** while **maintaining** its **immunogenic epitopes.**

A double mutant tat gene, constructed by D.Clements (Tulane University) was selected for these constructs.

This *tat* gene (originates from BH10 molecular clone) bears mutations in the active **site region (Lys41→Ala)and in RGD motif (Arg78→Lys and Asp80→Glu)** (Virology 235: 48-64, 1997).

The mutant tat gene was received as a cDNA fragment subcloned between the EcoRI and HindIII sites within a CMV expression plasmid (pCMVLys41/KGE)

### 2.1 CONSTRUCTION OF THE INTEGRATIVE VECTORS pRIT14912(encoding Tat mutant-His protein) and pRIT14913(encoding fusion Nef-Tat mutant-His).

The tat mutant gene was amplified by PCR from the pCMVLys41/KGE plasmid with primers 05 and 04 (see section 1.1 construction of pRIT14598)

An NcoI restriction site was introduced at the 5' end of the PCR fragment while a SpeI site was introduced at the 3' end with primer 04. The PCR fragment obtained and the PHII-D2-MOD vector were both restricted by NcoI and SpeI, purified on agarose gel and ligated to create the integrative plasmid pRIT14912

To construct pRIT14913, the tat mutant gene was amplified by PCR from the pCMVLys41/KGE plasmid with primers 03 and 04.

SpeI
PRIMER 03 (Seq ID NO 3): 5' ATCGTACTAGT.GAG.CCA.GTA.GAT.C 3'

SpeI

PRIMER 04 (Seq ID NO 4): 5' CGGCTACTAGTTTCCTTCGGGCCT 3'

The PCR fragment obtained and the plasmid pRIT 14597 (expressing Nef-His protein) were both digested by SpeI restriction enzyme, purified on agarose gel and ligated to create the integrative plasmid pRIT14913

### 2.2 TRANSFORMATION OF PICHIA PASTORIS STRAIN GS115.

Pichia pastoris strains expressing Tat mutant-His protein and the fusion Nef-Tat mutant-His were obtained, by applying integration and recombinant strain selection strategies previously described in section 1.2.

Two recombinant strains producing Tat mutant-His protein ,a 95 amino-acids protein, were selected: Y1775 (Mut⁺ phenotype) and Y1776(Mut^{s} phenotype).

One recombinant strain expressing Nef-Tat mutant-His fusion protein, a 302 amino-acids protein was selected: Y1774(Mut⁺ phenotype).

### Example 3: FERMENTATION OF PICHIA PASTORIS PRODUCING RECOMBINANT TAT-HIS.

A typical process is described in the table hereafter.

Fermentation includes a growth phase (feeding with a glycerol-based medium according to an appropriate curve) leading to a high cell density culture and an induction phase (feeding with a methanol and a salts/micro-elements solution). During fermentation the growth is followed by taking samples and measuring their absorbance at 620 nm. During the induction phase methanol was added via a pump and its concentration monitored by Gas chromatography (on culture samples) and by on-lin gas analysis with a Mass spectrometer. After fermentation the cells were recovered by centrifugation at 5020g during 30' at 2-8°C and the cell paste stored at - 20°C. For further work cell paste was thawed, resuspended at an OD (at 620 nm) of 150 in a buffer (Na2HPO4 pH7 50 mM, PMSF 5%, Isopropanol 4 mM) and disrupted by 4 passages in a DynoMill (room 0.6L, 3000 rpm, 6L/H, beads diameter of 0.40-0.70 mm).

For evaluation of the expression samples were removed during the induction, disrupted and analyzed by SDS-Page or Western blot. On Coomassie blue stained SDS-gels the recombinant Tat-his was clearly identified as an intense band presenting a maximal intensity after around 72-96H induction.

| | |
|---|---|
| Thawing of a Working seed vial | |
| ↓ | |
| Solid preculture 30°C, 14-16H | Synthetic medium: YNB + glucose + agar |
| ↓ | |
| Liquid preculture in two 2L erlenmeyer 30°C, 200 rpm | Synthetic medium: 2 x 400 ml YNB + glycerol Stop when OD > 1 (at 620 nm) |
| ↓ | |
| Inoculation of a 20L fermentor | 5L initial medium (FSC006AA) |
| | 3 ml antifoam SAG471 (from Witco) |
| | Set-points: Temperature : 30°C |
| | Overpressure: 0.3 barg |
| | Air flow: 20 Nl/min |
| | Dissolved 02: regulated > 40% |
| | pH : regulated at 5 by NH₄OH |
| ↓ | |
| Fed-batch fermentation: growth phase Duration around 40H | Feeding with glycerol-based medium FFB005AA Final OD between 200-500 OD (620 nm) |
| Fed-batch fermentation: induction phase Duration: up to 97H | Feeding with methanol and with a salt/micro-elements solution (FSE021AB). |
| ↓ | |
| Centrifugation | 5020g /30 min / 2-8°C |
| ↓ | |
| Recover cell paste and store at -20°C | |
| ↓ | |
| Thaw cells and resuspend at OD150 (620 nm) in buffer | Buffer: Na2HPO4 pH7 50 mM, PMSF 5%, Isopropanol 4 mM |
| ↓ | |
| Cell disruption in Dyno-mill 4 passages | Dyno-mill: (room 0.6L, 3000 rpm, 6L/H, beads diameter of 0.40-0.70 mm). |
| ↓ | |
| Transfer for extraction/purification | |

**Media used for fermentation:**

| **Solid preculture: (YNB + glucose + agar)** | | | | | |
|---|---|---|---|---|---|
| Glucose: | 10 g/l | Na2MoO4.2H2O: | 0.0002 g/l | Acide folique: | |
| KH2PO4: | 1 g/l | MnSO4.H20 | 0.0004 g/l | Inositol: | 0.064 g/l |
| MgSO4.7H2O | 0.5 g/l | H3BO3: | 0.0005 g/l | Pyridoxine: | 0.008 g/l |
| CaC12.2H2O: | 0.1 g/l | KI: | 0.0001 g/l | Thiamine: | 0.008 g/l |
| NaCl: | 0.1 g/l | CoCl2.6H20: | 0.00009 g/l | Niacine: | 0.000032 |
| g/l | | | | | |
| FeCl3.6H20: | 0.0002 g/l | Riboflavine: | 0.000016 g/l | Panthotenate Ca: | 0.008 g/l |
| CuSO4.5H2O: | 0.00004 g/l | Biotine: | 0.000064 g/l | Para-aminobenzoic acid: | |
| ZnSO4.7H2O | 0.0004 g/l | (NH4)2SO4 | 5 g/l | Agar | 18 g/l |

| **Liquid preculture,(YNB + glycerol)** | | | | | |
|---|---|---|---|---|---|
| Glycerol: | 2%(v/v) | Na2MoO4.2H2O: | 0.0002 g/l | Acide folique: | |
| KH2PO4: | 1 g/l | MnSO4.H2O: | 0.0004 g/l | Inositol: | 0.064 g/l |
| MgSO4.7H2O: | 0.5 g/l | H3BO3 | 0.0005 g/l | Pyridoxine: | 0.008 g/l |
| CaCl2.2H2O: | 0.1 g/l | KI: | 0.0001 g/l | Thiamine: | 0.008 g/l |
| NaCl: | 0.1 g/l | CoCl2.6H2O: | 0.00009 g/l | Niacine: | 0.000032 |
| g/l | | | | | |
| FeCl3.6H2O: | 0.0002 g/l | Riboflavine: | 0.000016 g/l | Panthoténate Ca: | 0.008 g/l |
| CuSO4.5H2O: | 0.00004 g/l | Biotine: | 0.000064 g/l | Para-aminobenzoic acid: | |
| ZnSO4.7H2O: | 0.0004 g/l | (NH4)2SO4: | 5 g/l | | |

| **Initial fermentor change: (FSC006AA)** | | | |
|---|---|---|---|
| (NH4)₂SO4: 6.4 g/l | | | |
| KH2PO4: 9 g/l | Na2MoO4.2H2O: | 2.04 mg/l | |
| MgSO4.7H2O: | 4.7 g/l | MnSO4.H2O: | 4.08 |
| mg/l | | | |
| CaCl2.2H2O: 0.94 g/l | H3BO3 | 5.1 mg/l | |
| FeCl3.6H2O: 10 mg/l | KI: | 1.022 mg/l | |
| HCl: 1.67 ml/l | CoCl2.6H2O: | 0.91mg/l | |
| CuSO4.5H2O: | 0.408 mg/l | NaCl: | 0.06 |
| g/l | | | |
| ZnSO4.7H2O:4.08 mg/l | Biotine: | 0.534 mg/l | |

| **Feeding solution used for growth phase (FFB005AA)** | | | |
|---|---|---|---|
| Glycérol: | 38.7 % v/v | Na2MoO4.2H2O: | 5.7 |
| mg/l | | | |
| MgSO4.7H2O: | 13 g/l | CuSO4.5H2O: | 1.13 mg/l |
| CaCl2.2H2O: | 2.6 g/l | CoCl2.6H2O: | 2.5 mg/l |
| FeCl3.6H2O: | 27.8mg/l | H3BO3 | 14.2 mg/l |
| ZnSO4.7H2O | 11.3 mg/l | Biotine: | 1.5 mg/l |
| MnSO4.H2O | 11.3 mg/l | KI: | 2.84mg/l |
| KH2PO4: | 24.93 g/l | NaCl: | 0.167 g/l |

| **Feeding solution of salts and micro-elements used during induction (FSE021AB):** | | | | |
|---|---|---|---|---|
| KH2PO4: | 45 g/l | Na2MoO4.2H2O: | 10.2mg/l | |
| MgSO4.7H2O | | 23.5 g/l | MnSO4.H2O: | 20.4 |
| mg/l | | | | |
| CaCl2.2H2O | 4.70 g/l | H3BO3 | 25.5 mg/l | |
| NaCl: | 0.3 g/l | KI: | 5.11 mg/l | |
| HCl: | 8.3ml/l | CoCl2.6H2O | 4.55mg/l | |
| CuSO4.5H2O: | | 2.04 mg/l | FeCl3.6H2O | 50.0 |
| mg/l | | | | |
| ZnS04.7H2O:20.4 | mg/l | Biotine: | 2.70 mg/l | |

### Example 4: PURIFICATION OF Nef-Tat-His FUSION PROTEIN (PICHIA PASTORIS)

The purification scheme has been developed from 146g of recombinant Pichia pastoris cells (wet weight) or 2L Dyno-mill homogenate OD 55. The chromatographic steps are performed at room temperature. Between steps, Nef-Tat positive fractions are kept overnight in the cold room (+4°C) ; for longer time, samples are frozen at -20°C.

| | |
|---|---|
| 146g of Pichia pastoris cells | |
| ↓ | |
| Homogenization | Buffer: 2L 50 mM PO₄ pH 7.0 |
| | final OD:50 |
| ↓ | |
| Dyno-mill disruption (4 passes) | |
| ↓ | |
| Centrifugation | JA10 rotor / 9500 rpm/ 30 min / room temperature |
| ↓ | |
| Dyno-mill Pellet | |
| ↓ | |
| Wash | Buffer: +2L 10 mM PO₄ pH 7.5 - 150mM - NaCl 0,5% empigen |
| (1h - 4°C) | |
| ↓ | |
| Centrifugation | JA10 rotor / 9500 rpm/ 30 min / room temperature |
| ↓ | |
| Pellet | |
| ↓ | |
| Solubilisation | Buffer: + 660ml 10 mM PO₄ pH 7.5 - 150mM NaCl - 4.0M GuHCl |
| (O/N - 4°C) | |
| ↓ | |
| Reduction | + 0,2M 2-mercaptoethanesulfonic |
| (4H - room temperature - in the dark) | acid, sodium salt (powder addition) / pH adjusted to 7.5 (with 0,5M NaOH solution) before incubation |
| ↓ | |
| reaction with iodoacetamide (carbamidomethylation) (1/2 h - room temperature - in the dark) | + 0,25M Iodoacetamide (powder addition) / pH adjusted to 7.5 (with 0,5M NaOH solution) before incubation |
| ↓ | |
| Immobilized metal ion affinity chromatography on Ni⁺⁺-NTA-Agarose | Equilibration buffer: 10 mM P0₄ pH 7.5 - 150mM NaCl 4.0M |
| (Qiagen - 30 ml of resin) | GuHCl |
| | Washing buffer: 1) Equilibration buffer |
| | 2) 10 mMPO₄ pH 7.5 - 150mM NaCl - 6M Urea |
| | 3) 10 mM PO₄ pH 7.5 - 150mM NaCl - 6M Urea - 25 mM Imidazol |
| | Elution buffer: 10 mM PO₄ pH 7.5 - 150mM NaCl 6M Urea - 0,5M |
| | Imidazol |
| ↓ | |
| Dilution | Down to an ionic strength of 18 mS/cm² |
| | Dilution buffer: 10 mM PO₄ pH 7.5 - 6M Urea |
| ↓ | |
| Cation exchange chromatography on SP Sepharose FF | Equilibration buffer: 10 mM PO₄ pH 7.5 - 150mM NaCl - 6.0M |
| (Pharmacia - 30 ml of resin) | Urea |
| | Washing buffer: 1) Equilibration buffer |
| | 2) 10 mM PO₄ pH 7.5 - 250mM NaCl - 6M Urea |
| | Elution buffer: 10 mM Borate pH 9.0 - 2M NaCl - 6M Urea |
| ↓ | |
| Concentration | up to 5 mg/ml |
| | 10kDa Omega membrane(Filtron) |
| ↓ | |
| Gel filtration chromatography on Superdex200 XK 16/60 | Elution buffer: 10 mM PO₄ pH 7.5 - 150mM NaCl - 6M Urea |
| (Pharmacia - 120 ml of resin) | 5 ml of sample / injection → 5 injections |
| ↓ | |
| Dialysis | Buffer: 10 mM PO₄ pH 6.8 - 150mM NaCl - 0,5M Arginin* |
| (O/N - 4°C) | |
| ↓ | |
| Sterile filtration | Millex GV 0,22µm |

| | |
|---|---|
| * ratio: 0,5M Arginin for a protein concentration of 1600µg/ml. | |

### Purity

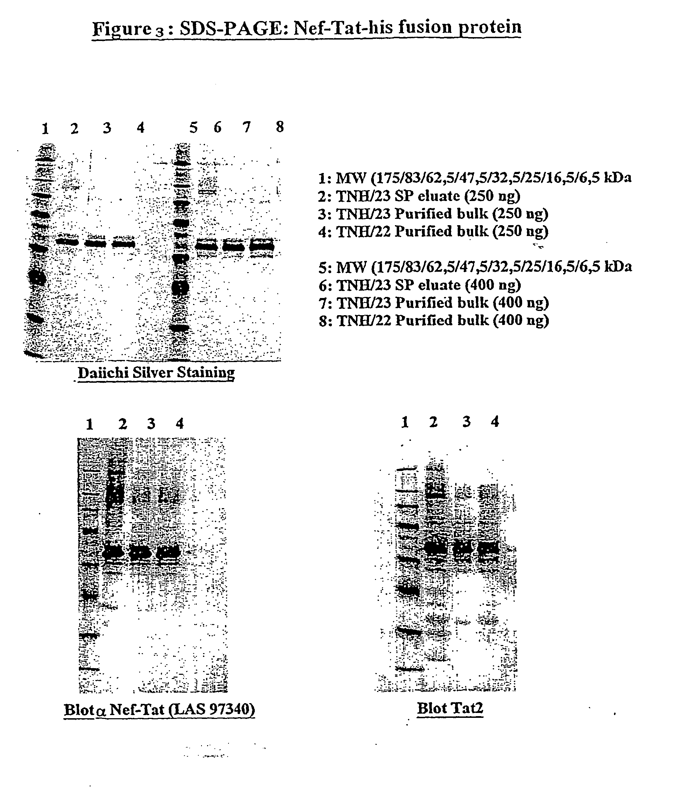

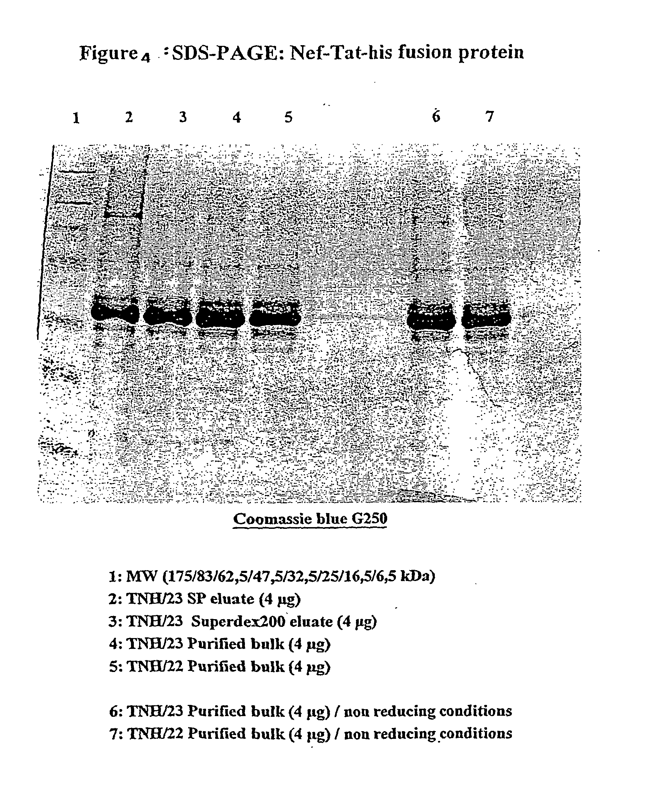

The level of purity as estimated by SDS-PAGE is shown in Figure 3 by Dauchi Silver Staining and in Figure 4 by Coomassie blue G250.
After Superdex200 step: > 95%
After dialysis and sterile filtration steps: > 95%

### Recovery

51mg of Nef-Tat-his protein are purified from 146g of recombinant Pichia pastoris cells (= 2L of Dyno-mill homogenate OD 55)

### Example 5: PURIFICATION OF OXIDIZED NEF-TAT-HIS FUSION PROTEIN IN PICHIA PASTORIS

The purification scheme has been developed from 73 g of recombinant Pichia pastoris cells (wet weight) or 1 L Dyno-mill homogenate OD 50. The chromatographic steps are performed at room temperature. Between steps , Nef-Tat positive fractions are kept overnight in the cold room (+4°C) ; for longer time, samples are frozen at -20°C.

| | |
|---|---|
| **73 g of Pichia pastoris cells** | |
| ↓ | |
| **Homogenization** | Buffer: 1L 50 mM PO₄ pH 7.0 - Pefabloc 5 mM |
| | final OD:50 |
| ↓ | |
| **Dyno-mill disruption (4 passes)** | |
| ↓ | |
| **Centrifugation** | JA10 rotor / 9500 rpm/ 30 min / room temperature |
| ↓ | |
| **Dyno-mill Pellet** | |
| ↓ | |
| **Wash** | Buffer: +1L 10 mM PO₄ pH 7.5 - 150 mM NaCl - 0,5% Empigen |
| **(2h - 4°C)** | |
| ↓ | |
| **Centrifugation** | JA10 rotor / 9500 rpm/ 30 min / room temperature |
| ↓ | |
| **Pellet** | |
| ↓ | |
| **Solubilisation** | Buffer: + 330ml 10 mM PO₄ pH 7.5 - |
| **(O/N - 4°C)** | 150mM NaCl - 4.0M GuHCl |
| ↓ | |
| **Immobilized metal ion affinity** | Equilibration buffer: 10 mM PO₄ pH 7.5 - 150 mM NaCl - 4.0 M GuHCI |
| **chromatography on Ni⁺⁺-NTA-Agarose** | |
| **(Qiagen - 15 ml of resin)** | Washing buffer: 1) Equilibration buffer |
| | 2) 10 mM PO₄ pH 7.5 - 150mMNaCl-6M |
| | Urea |
| | 3) 10 mM PO₄ pH 7.5 - 150 mM NaCl - 6M |
| | Urea - 25 mM Imidazol |
| | Elution buffer: 10 mM PO₄ pH 7.5 - 150 mM NaCl - 6 M Urea - 0,5 M Imidazol |
| ↓ | |
| **Dilution** | Down to an ionic strength of 18 mS/cm² Dilution buffer: 10 mM PO₄ pH 7.5 - 6 M Urea |
| ↓ | |
| **Cation exchange chromatography on SP** | Equilibration buffer: 10 mM PO₄ pH 7.5 - 150 mM NaCl - 6.0 M Urea |
| **Sepharose FF** | |
| **(Pharmacia - 7 ml of resin)** | Washing buffer: 1) Equilibration buffer |
| | 2) 10 mM PO₄ pH 7.5 - 250mM NaCl - 6M |
| | Urea |
| | Elution buffer: 10 mM Borate pH 9.0 - 2M NaCl - 6M Urea |
| ↓ | |
| **Concentration** | up to 0,8 mg/ml 10kDa Omega membrane(Filtron) |
| ↓ | |
| **Dialysis** | Buffer: 10 mM PO₄ pH 6.8 -150 mM |
| **(O/N - 4°C)** | NaCl - 0,5 M Arginin |
| ↓ | |
| **Sterile filtration** | Millex GV 0,22µm |

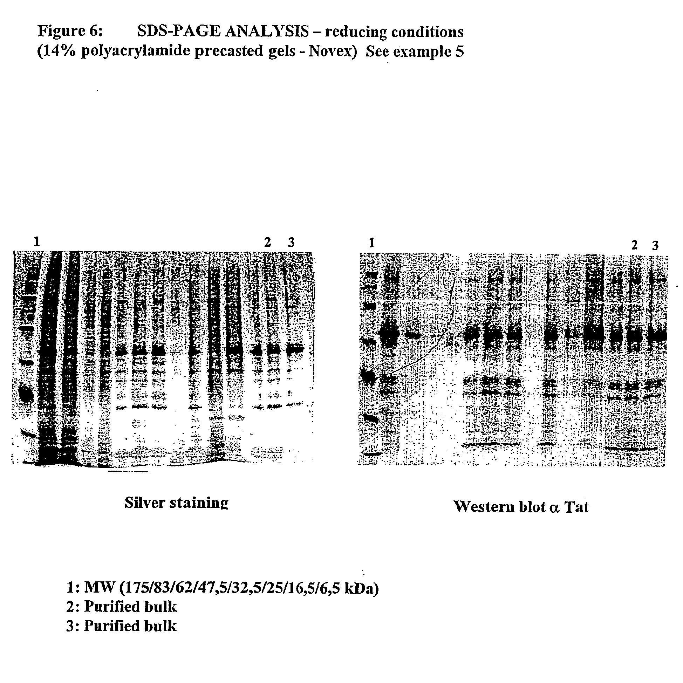

### → Level of purity estimated by SDS-PAGE is shown in Figure 6 (Daiichi Silver Staining, Coomassie blue G250, Western blotting):

After dialysis and sterile filtration steps: > 95%

### → Recovery (evaluated by a colorimetric protein assay: DOC TCA BCA)

2,8 mg of oxidized Nef-Tat-his protein are purified from 73 g of recombinant Pichia pastoris cells (wet weight) or 1 L of Dyno-mill homogenate OD 50.

### Example 6: PURIFICATION OF REDUCED TAT-HIS PROTEIN (PICHIA PASTORIS)

The purification scheme has been developed from 160 g of recombinant Pichia pastoris cells (wet weight) or 2L Dyno-mill homogenate OD 66. The chromatographic steps are performed at room temperature. Between steps, Tat positive fractions are kept overnight in the cold room (+4°C) ; for longer time, samples are frozen at -20°C.

| | |
|---|---|
| 160 g of Pichia pastoris cells | |
| ↓ | |
| Homogenization | Buffer: +2 L 50 mM PO₄ pH 7.0 - 4 mM PMSF final OD:66 |
| ↓ | |
| Dyno-mill disruption (4 passes) | |
| ↓ | |
| Centrifugation | JA10 rotor / 9500 rpm / 30 min / room temperature |
| ↓ | |
| Dyno-mill Pellet | |
| ↓ | |
| Wash | Buffer: +2 L 10 mM PO₄ pH 7.5 - 150 mM NaCl - 1% Empigen |
| (1h - 4°C) | |
| ↓ | |
| Centrifugation | JA10 rotor / 9500 rpm / 30 min / room temperature |
| ↓ | |
| Pellet | |
| ↓ | |
| Solubilisation | Buffer: + 660 ml 10 mM PO₄ pH 7.5 - 150 mM |
| (O/N - 4°C) | NaCl - 4.0 M GuHCl |
| ↓ | |
| Centrifugation | JA10 rotor / 9500 rpm / 30 min / room temperature |
| ↓ | |
| Reduction | + 0,2 M 2-mercaptoethanesulfonic acid, sodium salt (powder addition) / pH adjusted to 7.5 (with 1 M NaOH solution) before incubation |
| (4H - room temperature - in the dark) | |
| ↓ | |
| reaction with iodoacetamide | + 0,25 M Iodoacetamide (powder addition) / pH adjusted to 7.5 (with 1 M NaOH solution) before incubation |
| (1/2 h - room temperature - in the dark) | |
| ↓ | |
| Immobilized metal ion affinity | Equilibration buffer: 10 mM PO₄ pH 7.5 -150 mM |
| chromatography on Ni⁺⁺-NTA-Agarose | NaCl - 4.0 M GuHCl |
| (Qiagen - 60 ml of resin) | Washing buffer: 1) Equilibration buffer |
| | 2) 10 mM PO₄ pH 7.5 - 150 mM |
| | NaCl - 6M Urea |
| | 3) 10mM PO₄ pH7.5 - 150mM |
| | NaCl - 6M Urea - 35 mM |
| | Imidazol |
| | Elution buffer: 10 mM PO₄ pH 7.5 - 150 mM NaCl - 6 M Urea - 0,5 M Imidazol |
| ↓ | |
| Dilution | Down to an ionic strength of 12 mS/cm |
| | Dilution buffer: 20 mM Borate pH 8.5 - 6 M Urea |
| ↓ | |
| Cation exchange chromatography on SP Sepharose FF | Equilibration buffer: 20 mM Borate pH 8.5 - 150 mM NaCl - 6.0 M Urea |
| (Pharmacia - 30 ml of resin) | Washing buffer: Equilibration buffer |
| | Elution buffer: 20 mM Borate pH 8.5 - 400 mM NaCl - 6.0 M Urea |
| ↓ | |
| Concentration | up to 1,5 mg/ml |
| | 10kDa Omega membrane(Filtron) |
| ↓ | |
| Dialysis | Buffer: 10 mM PO₄ pH 6.8 - 150 mM NaCl - 0,5 M Arginin |
| (O/N - 4°C) | |
| ↓ | |
| Sterile filtration | Millex GV 0,22 µm |

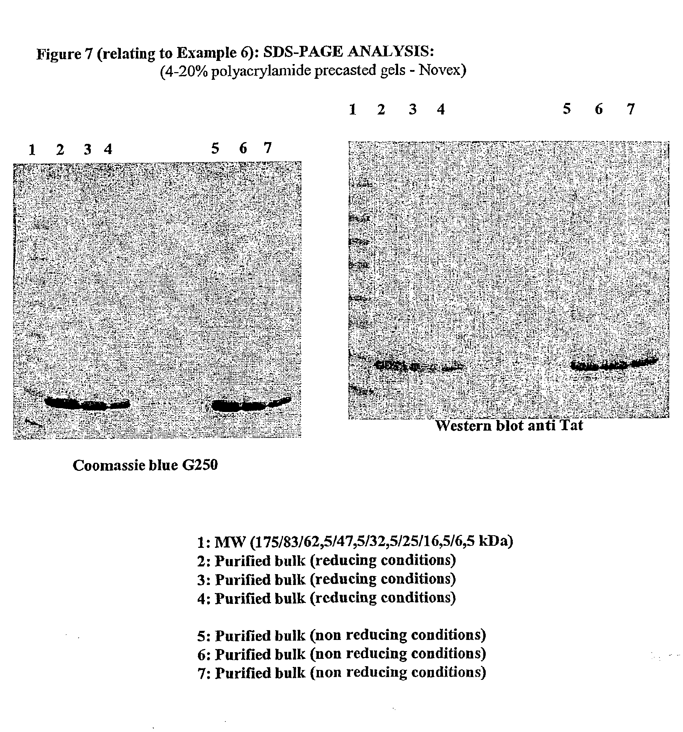

### → Level of purity estimated by SDS-PAGE as shown in Figure 7(Daiichi Silver Staining, Coomassie blue G250, Western blotting):

After dialysis and sterile filtration steps: > 95%

### → Recovery (evaluated by a colorimetric protein assay: DOC TCA BCA)

48 mg of reduced Tat-his protein are purified from 160 g of recombinant Pichia pastoris cells (wet weight) or 2 L of Dyno-mill homogenate OD 66.

### Example 7: Purification of oxidized Tat-his protein (Pichia Pastoris)

The purification scheme has been developed from 74 g of recombinant Pichia pastoris cells (wet weight) or 1L Dyno-mill homogenate OD60. The chromatographic steps are performed at room temperature. Between steps, Tat positive fractions are kept overnight in the cold room (+4°C) ; for longer time, samples are frozen at -20°C.

| | |
|---|---|
| 74 g of Pichia pastoris cells | |
| ↓ | |
| Homogenization | Buffer: +1 L 50 mM PO₄ pH 7.0 - 5 mM Pefabloc final OD:60 |
| ↓ | |
| Dyno-mill disruption (4 passes) | |
| ↓ | |
| Centrifugation | JA10 rotor / 9500 rpm / 30 min / room temperature |
| ↓ | |
| Dyno-mill Pellet | |
| ↓ | |
| Wash | Buffer:+1 L 10 mM PO₄ pH 7.5 -150 mM NaCl - 1% Empigen |
| (1h - 4°C) | |
| ↓ | |
| Centrifugation | JA10 rotor / 9500 rpm / 30 min / room temperature |
| ↓ | |
| Pellet | |
| ↓ | |
| Solubilisation | Buffer: + 330 ml 10 mM PO₄ pH 7.5 - 150 mM |
| (O/N - 4°C) | NaCl - 4.0 M GuHCl |
| ↓ | |
| Centrifugation | JA10 rotor / 9500 rpm / 30 min / room temperature |
| ↓ | |
| Immobilized metal ion affinity | Equilibration buffer: 10 mM PO₄ pH 7.5 -150 mM |
| chromatography on Ni⁺⁺-NTA-Agarose | NaCl - 4.0 M GuHCl |
| (Qiagen - 30 ml of resin) | Washing buffer: 1) Equilibration buffer |
| | 2) 10 mM PO₄ pH 7.5 - 150 mM |
| | NaCl - 6 M Urea |
| | 3) 10 mM PO₄ pH 7.5 - 150 mM |
| | NaCl - 6 M Urea - 35 mM |
| | Imidazol |
| | Elution buffer: 10 mM PO₄ pH 7.5 - 150 mM |
| | NaCl - 6 M Urea - 0,5 M Imidazol |
| ↓ | |
| Dilution | Down to an ionic strength of 12 mS/cm Dilution buffer: 20 mM Borate pH 8.5 - 6 M Urea |
| ↓ | |
| Cation exchange chromatography on SP | Equilibration buffer: 20 mM Borate pH 8.5 - |
| Sepharose FF | 150 mM NaCl - 6.0 M Urea |
| (Pharmacia - 15 ml of resin) | Washing buffer: 1) Equilibration buffer |
| | 2) 20 mM Borate pH 8.5 - |
| | 400 mM NaCl - 6.0 M Urea |
| | Elution buffer: 20 mM Piperazine pH 11.0 - 2 M NaCl - 6 M Urea |
| ↓ | |
| Concentration | up to 1,5 mg/ml 10 kDa Omega membrane(Filtron) |
| ↓ | |
| Dialysis | Buffer: 10 mM PO₄ pH 6.8 - 150 mM NaCl - |
| (O/N - 4°C) | 0,5 M Arginin |
| ↓ | |
| Sterile filtration | Millex GV 0,22 µm |

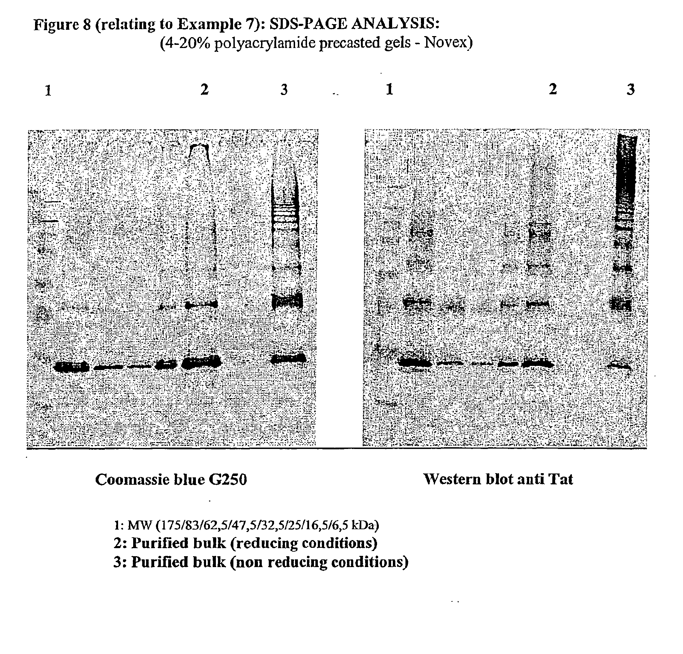

### → Level of purity estimated by SDS-PAGE as shown in Figure 8 (Daiichi Silver Staining, Coomassie blue G250, Western blotting):

After dialysis and sterile filtration steps: > 95%

### → Recovery (evaluated by a colorimetric protein assay: DOC TCA BCA)

19 mg of oxidized Tat-his protein are purified from 74 g of recombinant Pichia pastoris cells (wet weight) or 1 L of Dyno-mill homogenate OD 60.

### Example 8: PURIFICATION OF SIV REDUCED NEF-HIS PROTEIN (PICHIA PASTORIS)

The purification scheme has been developed from 340 g of recombinant Pichia pastoris cells (wet weight) or 4 L Dyno-mill homogenate OD 100. The chromatographic steps are performed at room temperature. Between steps , Nef positive fractions are kept overnight in the cold room (+4°C) ; for longer time, samples are frozen at -20°C.

| | |
|---|---|
| **340 g of Pichia pastoris cells** | |
| ↓ | |
| **Homogenization** | Buffer: 4L 50 mM PO₄ pH 7.0 - PMSF 4mM |
| | final OD:100 |
| ↓ | |
| **Dyno-mill disruption (4 passes)** | |
| ↓ | |
| **Centrifugation** | JA10 rotor / 9500 rpm/ 60 min / room temperature |
| ↓ | |
| **Dyno-mill Pellet** | |
| ↓ | |
| **Solubilisation** | Buffer: + 2,6 L 10 mM PO₄ pH 7.5 - |
| **(O/N - 4°C)** | 150mM NaCl - 4.0M GuHCl |
| ↓ | |
| **Centrifugation** | JA10 rotor / 9500 rpm / 30 min / room temperature |
| ↓ | |
| **Reduction** | + 0,2 M 2-mercaptoethanesulfonic acid, sodium salt (powder addition) / pH adjusted to 7.5 (with 1 M NaOH solution) before incubation |
| **(4H - room temperature - in the dark)** | |
| ↓ | |
| **Reaction with iodoacetamide** | + 0,25 M Iodoacetamide (powder addition) / pH adjusted to 7.5 (with 1 M NaOH solution) before incubation |
| **(1/2 h - room temperature - in the dark)** | |
| ↓ | |
| **Immobilized metal ion affinity** | Equilibration buffer: 10 mM PO₄ pH 7.5 |
| **chromatography on Ni⁺⁺-NTA-Agarose** | -150 mM NaCl- 4.0 M GuHCl |
| **(Qiagen - 40 ml of resin)** | Washing buffer: 1) Equilibration buffer |
| | 2) 10mM PO₄ pH7.5 - 150 mM NaCl - 6 M |
| | Urea - 25 mM Imidazol |
| | Elution buffer: 10 mM PO₄ pH 7.5 - 150 mM NaCl - 6 M Urea - 0,5 M |
| | Imidazol |
| ↓ | |
| **Concentration** | up to 3 mg/ml |
| | 10kDa Omega membrane(Filtron) |
| ↓ | |
| **Gel filtration chromatography on** | Elution buffer: 10 mM PO₄ pH 7.5 - 150 mM NaCl - 6 M Urea |
| **Superdex 200** | |
| **(Pharmacia - 120 ml of resin)** | |
| ↓ | |
| **Concentration** | up to 1,5 mg/ml |
| | 10kDa Omega membrane(Filtron) |
| ↓ | |
| **Dialysis** | Buffer: 10 mM PO₄ pH 6.8 - 150 mM |
| **(O/N - 4°C)** | NaCl - Empigen 0,3% |
| ↓ | |
| **Sterile filtration** | Millex GV 0,22µm |

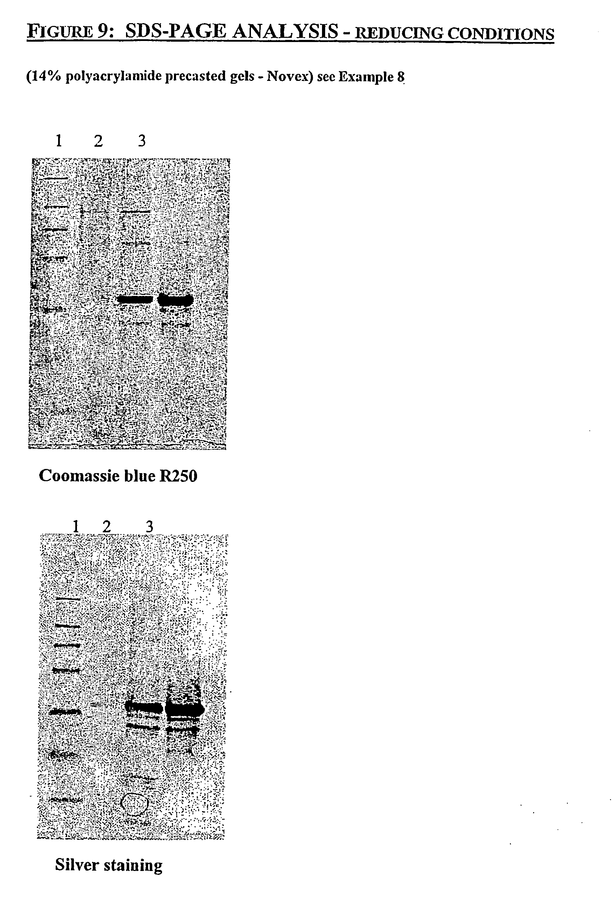

### → Level of purity estimated by SDS-PAGE as shown in Figure 9 (Daiichi Silver Staining, Coomassie blue G250, Western blotting):

After dialysis and sterile filtration steps: > 95%

### → Recovery (evaluated by a colorimetric protein assay: DOC TCA BCA)

20 mg of SIV reduced Nef -his protein are purified from 340 g of recombinant Pichia pastoris cells (wet weight) or 4 L of Dyno-mill homogenate OD 100.

### Example 9: PURIFICATION OF HIV REDUCED NEF-HIS PROTEIN (PICHIA PASTORIS)

The purification scheme has been developed from 160 g of recombinant Pichia pastoris cells (wet weight) or 3 L Dyno-mill homogenate OD 50. The chromatographic steps are performed at room temperature. Between steps , Nef positive fractions are kept overnight in the cold room (+4°C) ; for longer time, samples are frozen at -20°C.

| | |
|---|---|
| **160 g of Pichia pastoris cells** | |
| ↓ | |
| **Homogenization** | Buffer: 3 L 50 mM PO₄ pH 7.0 - Pefabloc 5 mM final OD:50 |
| ↓ | |
| **Dyno-mill disruption (4 passes)** | |
| ↓ | |
| **Freezing/Thawing** | |
| ↓ | |
| **Centrifugation** | JA10 rotor / 9500 rpm/ 60 min / room temperature |
| ↓ | |
| **Dyno-mill Pellet** | |
| ↓ | |
| **Solubilisation** | Buffer: + 1 L 10 mM PO₄ pH 7.5 - 150mM NaCl - 4.0M GuHCl |
| **(O/N - 4°C)** | |
| ↓ | |
| **Centrifugation** | JA10 rotor / 9500 rpm / 60 min / room temperature |
| ↓ | |
| **Reduction** | + 0,1 M 2-mercaptoethanesulfonic acid, sodium salt (powder addition) / pH adjusted to 7.5 (with 1 M NaOH solution) before incubation |
| **(3 H - room temperature - in the dark)** | |
| ↓ | |
| **Reaction with iodoacetamide** | + 0,15 M Iodoacetamide (powder addition) / pH adjusted to 7.5 (with 1 M NaOH solution) before incubation |
| **(1/2 h - room temperature - in the dark)** | |
| ↓ | |
| **Immobilized metal ion affinity** | Equilibration buffer: 10 mM PO₄ pH 7.5 |
| **chromatography on Ni⁺⁺-NTA-Agarose** | - 150 mM NaCl - 4.0 M GuHCl |
| **(Qiagen - 10 ml of resin)** | Washing buffer: 1) Equilibration buffer |
| | 2) 10 mM PO₄ pH 7.5 - 150 mM NaCl - 6 M Urea |
| | 3) 10 mM PO₄ pH 7.5 - 150 mM NaCl - 6 M Urea - 25 mM Imidazol |
| | Elution buffer: 10 mM Citrate pH 6.0 - 150 mM NaCl - 6 M Urea - 0,5 M |
| | Imidazol |
| ↓ | |
| **Concentration** | up to 3 mg/ml |
| | 10kDa Omega membrane(Filtron) |
| ↓ | |
| **Gel filtration chromatography on** | Elution buffer: 10 mM PO₄ pH 7.5 - 150 mM NaCl - 6 M Urea |
| **Superdex 200** | |
| **(Pharmacia -120 ml of resin)** | |
| ↓ | |
| **Dialysis** | Buffer: 10 mM PO₄ pH 6.8 -150 mM |
| **(O/N - 4°C)** | NaCl - 0,5M Arginin |
| ↓ | |
| **Sterile filtration** | Millex GV 0,22µm |

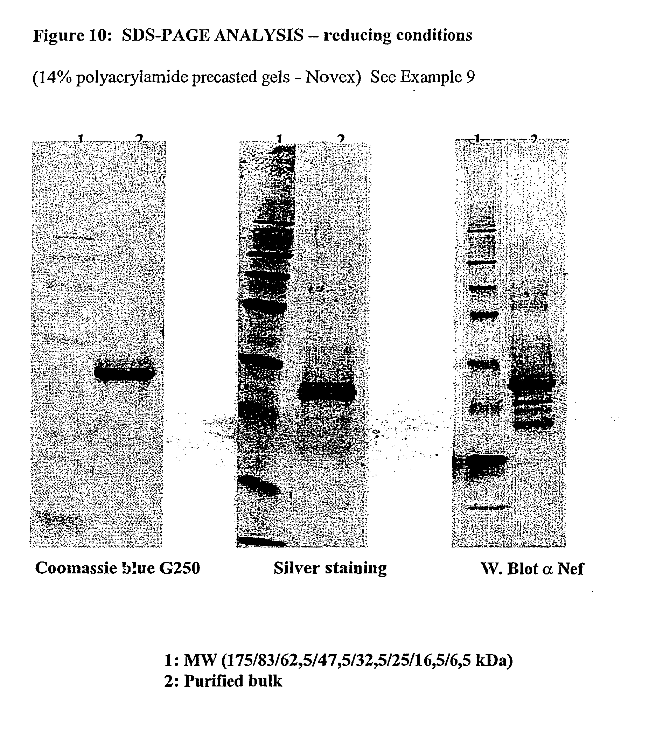

### → Level of purity estimated by SDS-PAGE as shown in Figure 10 (Daiichi Silver Staining, Coomassie blue G250, Western blotting):

After dialysis and sterile filtration steps: > 95%

### → Recovery (evaluated by a colorimetric protein assay: DOC TCA BCA)

20 mg of HIV reduced Nef-his protein are purified from 160 g of recombinant Pichia pastoris cells (wet weight) or 3 L of Dyno-mill homogenate OD 50.

### Example 10: EXPRESSION OF SIV nef SEQUENCE IN PICHIA PASTORIS

In order to evaluate Nef and Tat antigens in the pathogenic SHIV challenge model, we have expressed the Nef protein of simian immunodeficiency virus (SIV) of macaques, SIVmac239 ( Aids Research and Human Retroviruses, 6:1221-1231,1990). In the Nef coding region , SIV mac 239 has an in-frame stop codon after 92aa predicting a truncated product of only 10kD. The remainder of the Nef reading frame is open and would be predicted to encode a protein of 263aa (30kD) in its fully open form.

Our starting material for SIVmac239 *nef* gene was a DNA fragment corresponding to the complete coding sequence, cloned on the LX5N plasmid (received from Dr R.C. Desrosiers, Southborough,MA,USA).
This SIV *nef* gene is mutated at the premature stop codon (nucleotide G at position 9353 replaces the original T nucleotide) in order to express the full-length SIVmac239 Nef protein.

To express this SIV *nef gene* in *Pichia pastoris,* the PHIL-D2-MOD Vector (previously used for the expression of *HIV-1 nef* and *tat* sequences) was used. The recombinant protein is expressed under the control of the inducible alcohol oxidase (AOX1) promoter and the c-terminus of the protein is elongated by a Histidine affinity tail that will facilitate the purification.

### 10.1 CONSTRUCTION OF THE INTEGRATIVE VECTOR _{P}RIT 14908

To construct **pRIT 14908 ,** the SIV *nef gene* was amplified by PCR from the pLXSN/SIV-NEF plasmid with primers SNEF1 and SNEF2.

PRIMER SNEF1: 5' ATCGTCCATG.GGTGGAGCTATTTT 3'
NcoI
(Seq ID No: 6)

PRIMER SNEF2: 5' CGGCTACTAGTGCGAGTTTCCTT 3'
SpeI
(Seq ID No: 7)

The SIV *nef* DNA region amplified starts at nucleotide 9077 and terminates at nucleotide 9865 (Aids Research and Human Retroviruses, 6:1221-1231,1990).

An NcoI restriction site (with carries the ATG codon of the *nef* gene) was introduced at the 5' end of the PCR fragment while a SpeI site was introduced at the 3' end. The PCR fragment obtained and the integrative PHIL-D2-MOD vector were both restricted by NcoI and SpeI. Since one NcoI restriction site is present on the SIV *nef* amplified sequence (at position 9286), two fragments of respectively ±200bp and ± 600bp were obtained, purified on agarose gel and ligated to PHIL-D2-MOD vector. The resulting recombinant plasmid received, after verification of the *nef* amplified region by automated sequencing, the pRIT 14908 denomination.

### 10.2 TRANSFORMATION OF PICHIA PASTORIS STRAIN GS115(his4).

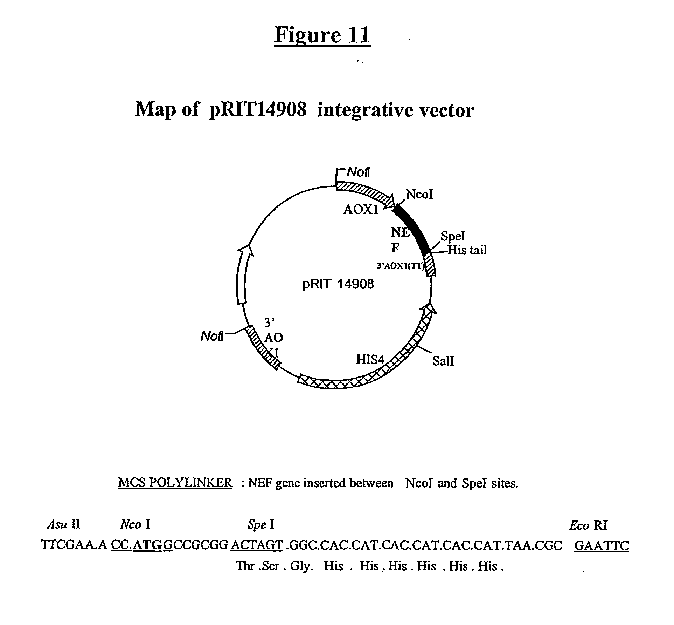

To obtain *Pichia pastoris* strain expressing SIV *nef*-His, strain GS115 was transformed with a linear NotI fragment carrying only the expression cassette and the HIS4 gene (Fig.11).

This linear NotI DNA fragment ,with homologies at both ends with AOX1 resident *P.pastoris* gene, favors recombination at the AOX1 locus.

Multicopy integrant clones were selected by quantitative dot blot analysis.

One transformant showing the best production level for the recombinant protein was selected and received the **Y1772** denomination.

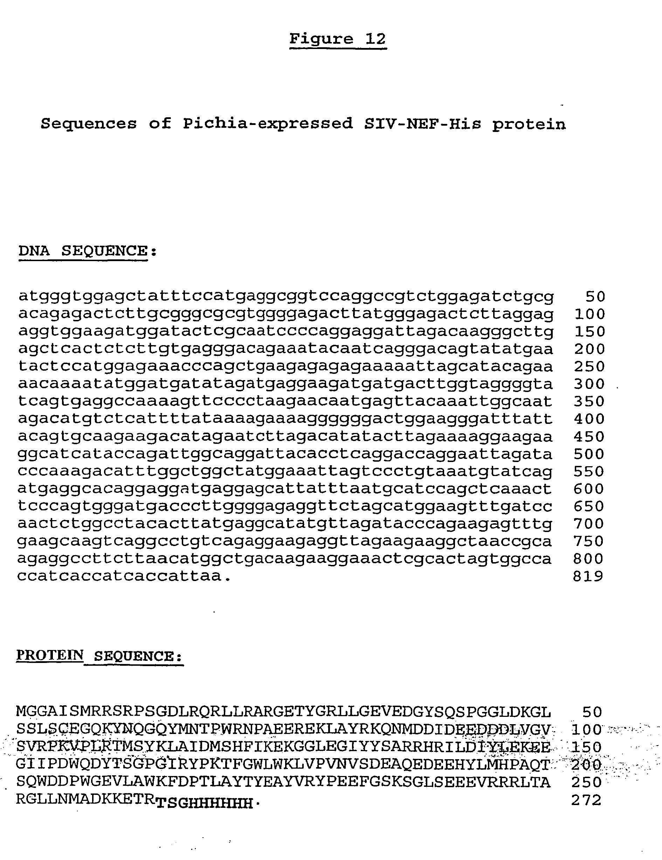

Strain **Y1772** produces the recombinant **SIV Nef-His** protein, a **272 amino acids** protein which would be composed of:
°Myristic acid
°A methionine, created by the use of NcoI cloning site of PHIL-D2-MOD vector.
°262 amino acids (aa) of Nef protein (starting at aa 2 and extending to aa 263, see Figure 12)
°A threonine and a serine created by the cloning procedure (cloning at SpeI site of PHIL-D2-MOD vector (Fig.11 - includes Seq ID No: 29).
°One glycine and six histidines.

Nucleic and Protein sequences are shown in Figure 12 (Seq ID Nos: 30 and 31).

### 10.3 CHARACTERIZATION OF THE EXPRESSED PRODUCT OF STRAIN Y1772.

### Expression level

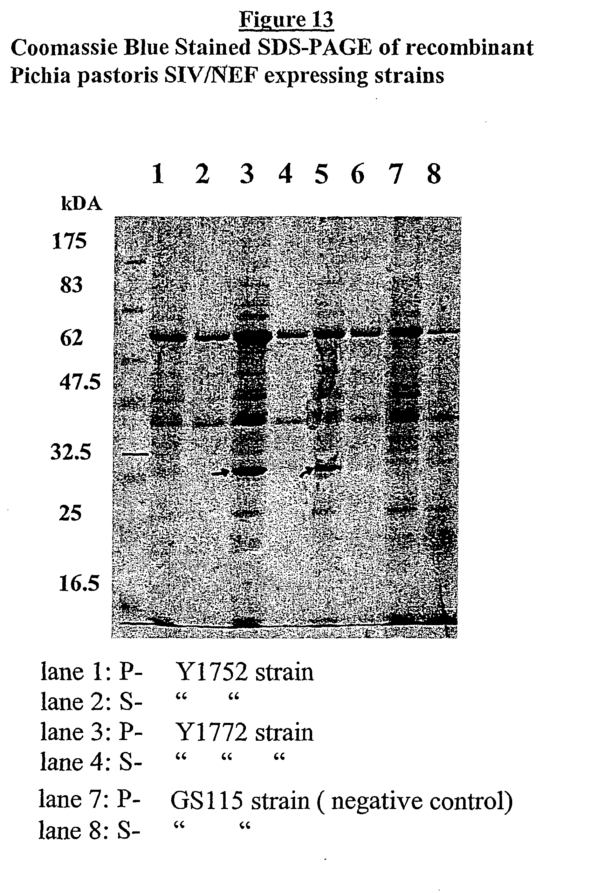

After 16 hours induction in medium containing 1% methanol as carbon source, abundance of the recombinant Nef-His protein, was estimated at 10% of total protein (Fig.13, lanes 3-4).

### Solubility

Induced cultures of recombinant strain Y1772 producing the Nef-His protein were centrifuged. Cell pellets were resuspended in breaking buffer, disrupted with 0.5mm glass beads and the cell extracts were centrifuged. The proteins contained in the insoluble pellet (P) and in the soluble supernatant (S) were compared on a Coomassie Blue stained SDS-PAGE10%.

As shown in figure 13, the majority of the recombinant protein from strain Y1772 (lanes 3-4) is associated with the insoluble fraction.

Strain Y1772 which presents a satisfactory recombinant protein expression level is used for the production and purification of SIV Nef-His protein.

### Example 11: EXPRESSION OF GP120 IN CHO

A stable CHO-K1 cell line which produces a recombinant gP120 glycoprotein has been established. Recombinant gP120 glycoprotein is a recombinant truncated form of the gP120 envelope protein of H1V-1 isolate W61D. The protein is excreted into the cell culture medium, from which it is subsequently purified.

### Construction of gp120 transfection plasmid pRIT13968

The envelope DNA coding sequence (including the 5'exon of tat and rev) of HIV-1 isolate W61D was obtained (Dr. Tersmette, CCB, Amsterdam) as a genomic gp160 envelope containing plasmid W61D (Nco-XhoI). The plasmid was designated pRIT13965.

In order to construct a gp120 expression cassette a stop codon had to be inserted at the amino acid glu 515 codon of the gp160 encoding sequence in pRIT13965 using a primer oligonucleotide sequence (DIR 131) and PCR technology. Primer DIR 131 contains three stop codons (in all open reading frames) and a SalI restriction site.

The complete gp120 envelope sequence was then reconstituted from the N-terminal BamH1-DraI fragment (170 bp) of a gp160 plasmid subclone pW61d env (pRIT13966) derived from pRIT13965, and the DraI-SalI fragment (510 bp) generated by PCR from pRIT13965. Both fragments were gel purified and ligated together into the E.coli plasmid pUC18, cut first by SalI (klenow treated), and then by BamH1. This resulted in plasmid pRIT13967. The gene sequence of the XmaI-SalI fragment (1580 bp) containing the gp120 coding cassette was sequenced and found to be identical to the predicted sequence. Plasmid RIT13967 was ligated into the CHO GS-expression vector pEE14 (Celltech Ltd., UK) by cutting first with BclI (klenow treated) and then by XmaI. The resulting plasmid was designated pRIT13968.

### Preparation of Master Cell Bank

The gp120-construct (pRIT13968) was transfected into CHO cells by the classical CaPO₄-precipitation/glycerol shock procedure. Two days later the CHOK1 cells were subjected to selective growth medium (GMEM + methionine sulfoximine (MSX) 25 µM + Glutamate + asparagine + 10% Foetal calf serum). Three chosen transfectant clones were further amplified in 175m² flasks and few cell vials were stored at -80°C. C-env 23,9 was selected for further expansion.

A small prebank of cells was prepared and 20 ampoules were frozen. For preparation of the prebank and the MCB, cells were grown in GMEM culture medium, supplemented with 7.5 % fetal calf serum and containing 50 µM MSX. These cell cultures were tested for sterility and mycoplasma and proved to be negative.

The Master Cell Bank CHOK1 env 23.9 (at passage 12) was prepared using cells derived from the premaster cell bank. Briefly, two ampoules of the premaster seed were seeded in medium supplemented with 7.5% dialysed foetal bovine serum. The cells were distributed in four culture flasks and cultured at 37°C. After cell attachment the culture medium was changed with fresh medium supplemented with 50 µM MSX. At confluence, cells were collected by trypsination and subcultured with a 1/8 split ratio in T-flasks - roller bottle - cell factory units. Cells were collected from cell factory units by trypsination and centrifugation. The cell pellet was resuspended in culture medium supplemented with DMSO as cryogenic preservative. Ampoules were prelabelled, autoclaved and heat-sealed (250 vials). They were checked for leaks and stored overnight at -70°C before storage in liquid nitrogen.

### Cell Culture And Production Of Crude Harvest

Two vials from a master cell bank are thawed rapidly. Cells are pooled and inoculated in two T-flasks at 37° ± 1°C with an appropriate culture medium supplemented with 7.5 % dialysed foetal bovine (FBS) serum. When reaching confluence (passage 13), cells are collected by trypsinisation, pooled and expanded in 10 T-flasks as above. Confluent cells (passage 14) are trypsinised and expanded serially in 2 cell factory units (each 6000 cm²; passage 15), then in 10 cell factories (passage 16). The growth culture medium is supplemented with 7.5 % dialysed foetal bovine (FBS) serum and 1% MSX. When cells reach confluence, the growth culture medium is discarded and replaced by "production medium" containing only 1 % dialysed foetal bovine serum and no MSX. Supernatant is collected every two days (48 hrs-interval) for up to 32 days. The harvested culture fluids are clarified immediately through a 1.2-0.22 µm filter unit and kept at -20°C before purification.

### Example 12: PURIFICATION OF HIV GP 120 (W61D CHO) FROM CELL CULTURE FLUID

All purification steps are performed in a cold room at 2-8°C. pH of buffers are adjusted at this temperature and are filtered on 0.2 µm filter. They are tested for pyrogen content (LAL assay). Optical density at 280 nm, pH and conductivity of column eluates are continuously monitored.

### (i) Clarified Culture Fluid

The harvested clarified cell culture fluid (CCF) is filter-sterilized and Tris buffer, pH 8.0 is added to 30 mM final concentration. CCF is stored frozen at -20°C until purification.

### (ii) Hydrophobic Interaction Chromatography

After thawing, ammonium sulphate is added to the clarified culture fluid up to 1 M. The solution is passed overnight on a TSK/TOYOPEARL-BUTYL 650 M (TOSOHAAS) column, equilibrated in 30 mM Tris buffer- pH 8.0 - 1 M ammonium sulphate. Under these conditions, the antigen binds to the gel matrix. The column is washed with a decreasing stepwise ammonium sulphate gradient. The antigen is eluted at 30 mM Tris buffer- pH 8.0 - 0.25 M ammonium sulphate.

### (iii) Anion-exchange Chromatography

After reducing the conductivity of the solution between 5 and 6 mS/cm, the gP120 pool of fractions is loaded onto a Q-sepharose Fast Flow (Pharmacia) column, equilibrated in Tris-saline buffer - pH 8.0. The column is operated on a negative mode, i.e. gP120 does not bind to the gel, while most of the impurities are retained.

### (iv) Concentration and diafiltration by ultrafiltration

In order to increase the protein concentration, the gP120 pool is loaded on a FILTRON membrane "Omega Screen Channel", with a 50 kDa cut-off. At the end of the concentration, the buffer is exchanged by diafiltration with 5 mM phosphate buffer containing CaCl₂ 0.3 mM, pH 7.0. If further processing is not performed immediately, the gP120 pool is stored frozen at -20°C. After thawing the solution is filtered onto a 0.2 µM membrane in order to remove insoluble materiel.

### (v) Chromatography on hydroxyapatite

The gP120 UF pool is loaded onto a macro-Prep Ceramic Hydroxyapatite, type II (Biorad) column equilibrated in 5 mM phosphate buffer + CaCl₂ 0.3 mM, pH 7.0. The column is washed with the same buffer. The antigen passes through the column and impurities bind to the column.

### (vi) Cation exchange chromatography

The gP120 pool is loaded on a CM/TOYOPEARL-650 S (TOSOHAAS) column equilibrated in acetate buffer 20 mM, pH 5.0. The column is washed with the same buffer, then acetate 20 mM, pH 5.0 and NaCl 10 mM. The antigen is then eluted by the same buffer containing 80 mM NaCl.

### (vii) Ultrafiltration

In order to augment the virus clearance capacity of the purification process, an additional ultrafiltration step is carried out. The gP120 pool is subjected to ultrafiltration onto a FILTRON membrane "Omega Screen Channel", cut-off 150 kDa. This pore-size membrane does not retain the antigen. After the process, the diluted antigen is concentrated on the same type of membrane (Filtron) but with a cut-off of 50 kDa.

### (viii) Size exclusion Gel Chromatography

The gP120 pool is applied to a SUPERDEX 200 (PHARMACIA) column in order to exchange the buffer and to eliminate residual contaminants. The column is eluted with phosphate buffer saline (PBS).

### (ix) Sterile filtration and storage

Fractions are sterilized by filtration on a 0.2 µM PVDF membrane (Millipore). After sterile filtration, the purified bulk is stored frozen at -20°C up to formulation. The purification scheme is summarized by the flow sheet below.
⇒ Level of purity of the purified bulk estimated by SDS-PAGE analysis (Silver staining / Coomassie Blue / Western Blotting) is ≥ 95%.
⇒ Production yield is around 2.5 mg /L CCF (according to Lowry assay) - Global purification yield is around 25% (according to Elisa assay)
⇒ Purified material is stable 1 week at 37°C (according to WB analysis)

Purification of gp120 from culture fluid

Mark √ indicate steps that are critical for virus removal.

| | |
|---|---|
| CLARIFIED CULTURE FLUID | |
| ↓ | |
| HYDROPHOBIC INTERACTION CHROMATOGRAPHY (BUTYL -TOYOPEARL 650 M) | |
| ↓ | |
| ANION EXCHANGE CHROMATOGRAPHY (NEGATIVE MODE) (Q-SEPHAROSE) | √ |
| ↓ | |
| 50 KD ULTRAFILTRATION (CONCENTRATION AND BUFFER EXCHANGE) | |
| ↓ | |
| (STORAGE -20°C) | |
| ↓ | |
| HYDROXYAPATITE CHROMATOGRAPHY (NEGATIVE MODE) (MACROPREP CERAMIC HYDROXYAPATITE II) | |
| ↓ | |
| CATION EXCHANGE CHROMATOGRAPHY (CM-TOYOPEARL 650 S) | |
| | |
| 150 KD ULTRAFILTRATION (OMEGA MEMBRANES / FILTRON) | √ |
| ↓ | |
| 50 KD ULTRAFILTRATION (CONCENTRATION) | |
| ↓ | |
| SIZE EXCLUSION CHROMATOGRAPHY (SUPERDEX 200) STERILE FILTRATION | √ |
| ↓ | |
| **PURIFIED BULK** | |
| STORAGE -20°C | |

### Example 13: VACCINE PREPARATION

A vaccine prepared in accordance with the invention comprises the expression products of one or more DNA recombinants encoding an antigen. Furthermore, the formulations comprise a mixture of 3 de -O-acylated monophosphoryl lipid A 3D-MPL and QS21 in an oil/water emulsion or an oligonucleotide containing umnethylated CpG dinucleotide motifs and aluminium hydroxide as carrier.

**3D-MPL:** is a chemically detoxified form of the lipopolysaccharide (LPS) of the Gram-negative bacteria Salmonella minnesota.

Experiments have shown that 3D-MPL combined with various vehicles strongly enhances both the humoral immunity and a T_{HI} type of cellular immunity.

**QS21:** is a saponin purified from a crude extract of the bark of the Quillaja Saponaria Molina tree, which has a strong adjuvant activity: it induces both antigen-specific lymphoproliferation and CTLs to several antigens.

Experiments have demonstrated a clear synergistic effect of combinations of 3D-MPL and QS21 1 in the induction of both humoral and T_{HI} type cellular immune responses.

**The oil/water emulsion** is composed of 2 oils (a tocopherol and squalene), and of PBS containing Tween 80 as emulsifier. The emulsion comprises 5% squalene, 5% tocopherol, 2% Tween 80 and has an average particle size of 180 nm (see WO 95/17210).

Experiments performed have proven that the adjunction of this O/W emulsion to 3D-MPL/QS21 further increases their immunostimulant properties.

### Preparation of the oil/water emulsion (2 fold concentrate)

Tween 80 is dissolved in phosphate buffered saline (PBS) to give a 2% solution in the PBS. To provide 100ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M110S Microfluidics machine. The resulting oil droplets have a size of approximately 180 nm.

### Preparation of oil in water formulation.

Antigens (100 µg gp120, 20 µg NefTat, and 20 µg SIV Nef, alone or in combination) were diluted in 10 fold concentrated PBS pH 6.8 and H₂O before consecutive addition of the oil in water emulsion, 3D-MPL (50µg), QS21 (50µg) and 1 ug/ml thiomersal as preservative at 5 min interval. The emulsion volume is equal to 50% of the total volume (250µl for a dose of 500µl).

All incubations were carried out at room temperature with agitation.

CpG oligonucleotide (CpG) is a synthetic unmethylated oligonucleotide containing one or several CpG sequence motifs. CpG is a very potent inducer of T_{HI} type immunity compared to the oil in water formulation that induces mainly a mixed T_{HI}/T_{H2} response. CpG induces lower level of antibodies than the oil in water formulation and a good cell mediated immune response. CpG is expected to induce lower local reactogenicity.

Preparation of CpG oligonucleotide solution: CpG dry powder is dissolved in H₂O to give a solution of 5 mg/ml CpG.

### Preparation of CpG formulation.

The 3 antigens were dialyzed against NaCl 150 mM to eliminate the phosphate ions that inhibit the adsorption of gp120 on aluminium hydroxide.

The antigens diluted in H₂O (100 µg gp120, 20 µg NefTat and 20 µg SIV Nef) were incubated with the CpG solution (500 µg CpG) for 30 min before adsorption on Al(OH)₃ to favor a potential interaction between the His tail of NefTat and Nef antigens and the oligonucleotide (stronger immunostimulatory effect of CpG described when bound to the antigen compared to free CpG). Then were consecutively added at 5 min interval Al(OH)₃ (500 µg), 10 fold concentrated NaCl and 1 µg/ml thiomersal as preservative.

All incubations were carried out at room temperature with agitation.

### Example 14: IMMUNIZATION AND SHIV CHALLENGE EXPERIMENT IN RHESUS MONKEYS.

### First Study

Groups of 4 rhesus monkeys were immunized intramuscularly at 0, 1 and 3 months with the following vaccine compositions:

| | | | | |
|---|---|---|---|---|
| Group 1: | Adjuvant 2 | + gp120 | | |
| Group 2: | Adjuvant 2 | + gp120 | + NefTat | + SIV Nef |
| Group 3: | Adjuvant 2 | | + NefTat* | + SIV Nef |
| Group 4 | Adjuvant 6 | + gp120 | + NefTat | + SIV Nef |
| Group 5 | Adjuvant 2 | | + NefTat | + SIV Nef |
| Group 6 | Adjuvant 2 | | | |

Adjuvant 2 comprises squalene/tocopherol/Tween 80/3D-MPL/QS21 and Adjuvant 6 comprises alum and CpG.

**Tat*** represents mutated Tat, in which Lys41→Ala and in RGD motif Arg78→Lys and Asp80→Glu ( Virology 235: 48-64, 1997).

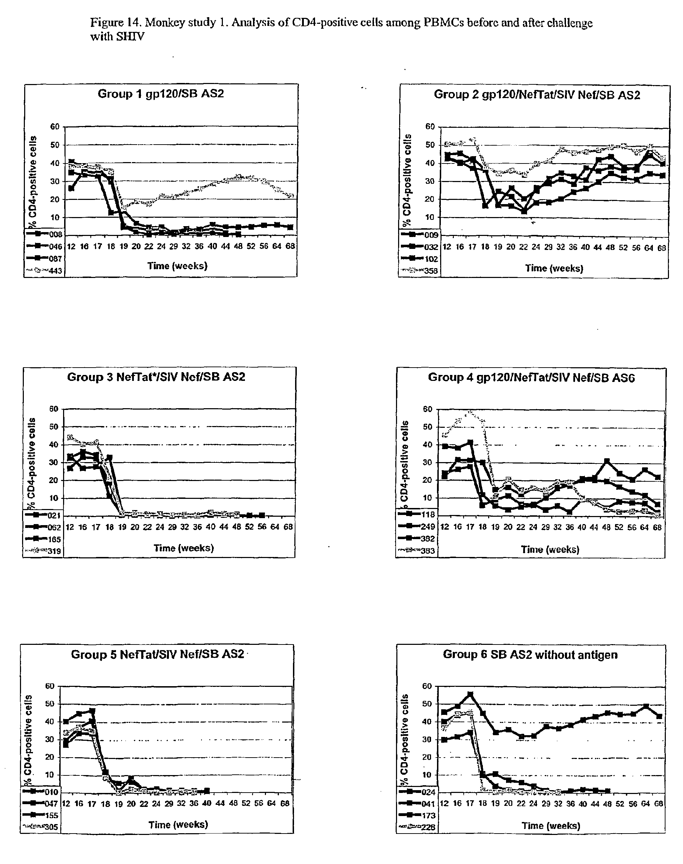

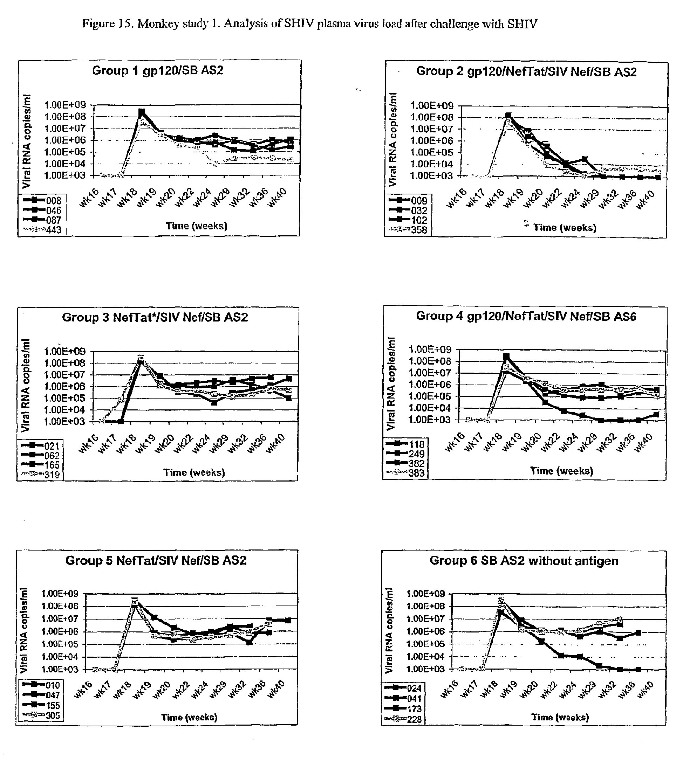

One month after the last immunization all animals were challenged with a pathogenic SHIV (strain 89.6p). From the week of challenge (wk16) blood samples were taken periodically at the indicated time points to determine the % of CD4-positive cells among peripheral blood mononuclear cells by FACS analysis (Figure 14) and the concentration of RNA viral genomes in the plasma by bDNA assay (Figure 15).

### Results

All animals become infected after challenge with SHIV_{89.6p}.

CD4-positive cells decline after challenge-in all animals of groups 1, 3, 5 and 6 except one animal in each of groups 1 and 6 (control group). All animals in group 2 exhibit a slight decrease in CD4-positive cells and recover to baseline levels over time. A similartrend is observed in group 4 animals (Figure 14).

Virus load data are almost the inverse of CD4 data. Virus load declines below the level of detection in 3/4 group 2 animals (and in the one control animal that maintains its CD4-positive cells), and the fourth animal shows only marginal virus load. Most of the other animals maintain a high or intermediate virus load (Figure 15).

Surprisingly, anti-Tat and anti-Nef antibody titres measured by ELISA were 2 to 3-fold higher in Group 3 (with mutated Tat) than in Group 5 (the equivalent Group with non-mutated Tat) throughout the course of the study.

At week 68 (56 weeks post challenge) all animals from the groups that had received the full antigen combination (groups 2 and 4) were still alive, while most of the animals in the other groupshad to be euthanized due to AIDS-like symptoms. The surviving animals per group were:

| | |
|---|---|
| Group 1: | 2/4 |
| Group 2: | 4/4 |
| Group 3: | 0/4 |
| Group 4 | 4/4 |
| Group 5 | 0/4 |
| Group 6 | 1/4 |

### Conclusions

The combination of gp120 and NefTat (in the presence of SIV Nef) prevents the loss of CD4-positive cells, reduces the virus load in animals infected with pathogenic

SHIV_{89.6p}, and delays or prevents the development of AIDS-like disease symptoms, while gp120 or NefTat/SIV Nef alone do not protect from the pathologic consequences of the SHIV challenge.

The adjuvant 2 which is an oil in water emulsion comprising squalene, tocopherol and Tween 80, together with 3D-MPL and QS21 seems to have a stronger effect on the study endpoints than the alum / CpG adjuvant.

### Second study

A second rhesus monkey SHIV challenge study was conducted to confirm the efficacy of the candidate vaccine gp120/NefTat + adjuvant and to compare different Tat-based antigens. The study was conducted by a different laboratory.

The design of the study was as follows.

Groups of 6 rhesus monkeys were immunized at 0, 4 and 12 weeks with injections i.m. and challenged at week 16 with a standard dose of pathogenic SHIV_{89.6p}.

Group 1 is the repeat of Group 2 in the first study.

| | | | | |
|---|---|---|---|---|
| Group 1: | Adjuvant 2 | + gp120 | +NefTat | + SIV Nef |
| Group 2: | Adjuvant 2 | + gp120 | + Tat (oxidised) | |
| Group 3: | Adjuvant 2 | + gp120 | + Tat (reduced) | |
| Group 4 | Adjuvant 2 | | | |

The follow-up/endpoints were again % CD4-positive cells, virus load by RT-PCR, morbidity and mortality

### Results

All animals except one in group 2 become infected after challenge with SHIV₈₉.₆ₚ.

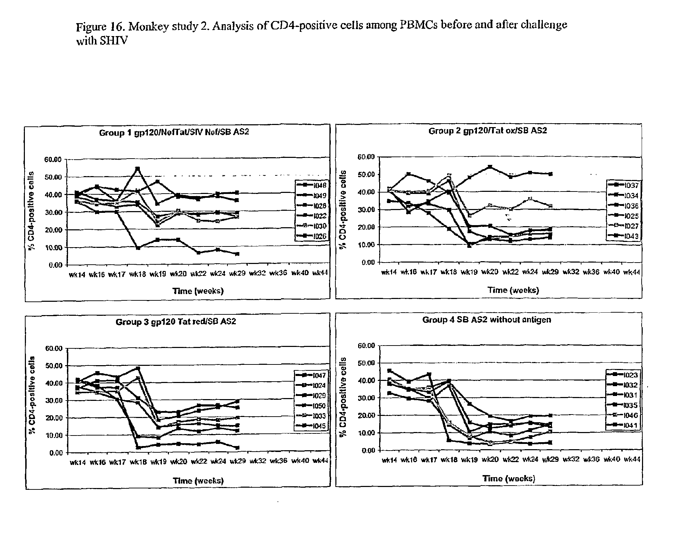

CD4-positive cells decline significantly after challenge in all animals of control group 4 and group 3, and in all but one animals of group 2. Only one animal in group 1 shows a marked decrease in CD4-positive cells. Unlike the animals from the first study, the monkeys in the second experiment display a stabilisation of CD4-positive cells at different levels one month after virus challenge (Figure 16). The stabilisation is generally lower than the initial % of CD4-positive cells, but will never lead to a complete loss of the cells. This may be indicative of a lower susceptibility to SHIV-induced disease in the monkey population that was used for the second study. Nonetheless, a beneficial effect of the gp120/NefTat/SIV Nef vaccine and the two gp120/Tat vaccines is demonstrable. The number of animals with a % of CD4-positive cells above 20 is 5 for the vaccinated animals, while none of the control animals from the adjuvant group remains above that level.

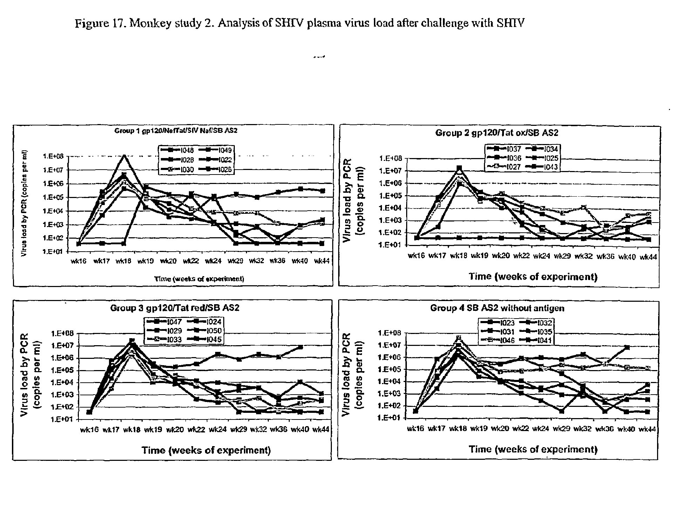

Analysis of RNA plasma virus loads confirms the relatively low susceptibility of the study animals (Figure 17). Only 2 of the 6 control animals maintain a high virus load, while the virus disappears from the plasma in the other animals. Thus, a vaccine effect is difficult to demonstrate for the virus load parameter.

### Conclusions

Analysis of CD4-positive cells indicates that the vaccine gp120/NefTat + adjuvant (in the presence of SIV Nef) prevents the drop of CD4-positive cells in most vaccinated animals This is a confirmation of the result obtained in the first SHIV study. Due to the lack of susceptibility of the study animals, the virus load parameter could not be used to demonstrate a vaccine effect. Taken together, the combination of gp120 and Tat and Nef HIV antigens provides protection against the pathologic consequences of HIV infection, as evidenced in a SHIV model.

The Tat alone antigens in combination with gp120 also provide some protection from the decline of CD4-positive cells. The effect is less pronounced than with the gp120/NefTat/SIV Nef antigen combination, but it demonstrates that gp120 and Tat are able to mediate some protective efficacy against SHIV-induced disease manifestations.

The second SHIV challenge study was performed with rhesus monkeys from a source completely unrelated to the source of animals from the first study. Both parameters, % of CD4-positive cells and plasma virus load, suggest that the animals in the second study were less susceptible to SHIV-induced disease, and that there was considerably greater variability among the animals. Nonetheless, a beneficial effect on the maintenance of CD4-positive cells of the gp120/NefTat/SIV Nef vaccine was seen with the experimental vaccine containing gp120/NefTat and SIV Nef. This indicates that the vaccine effect was not only repeated in a separate study, but furthermore demonstrated in an unrelated monkey population.

### Example 15: Generation of PMID vectors for gp120 and Nef/Tat

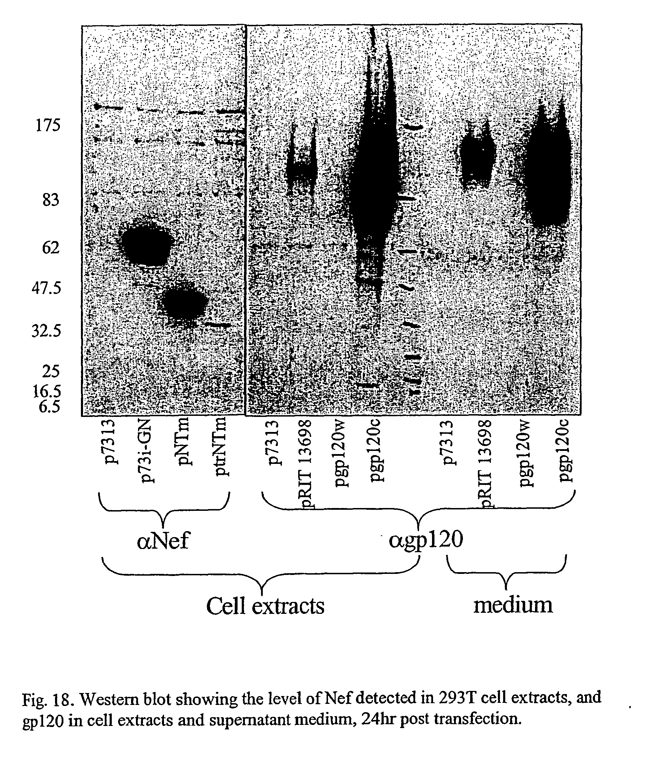

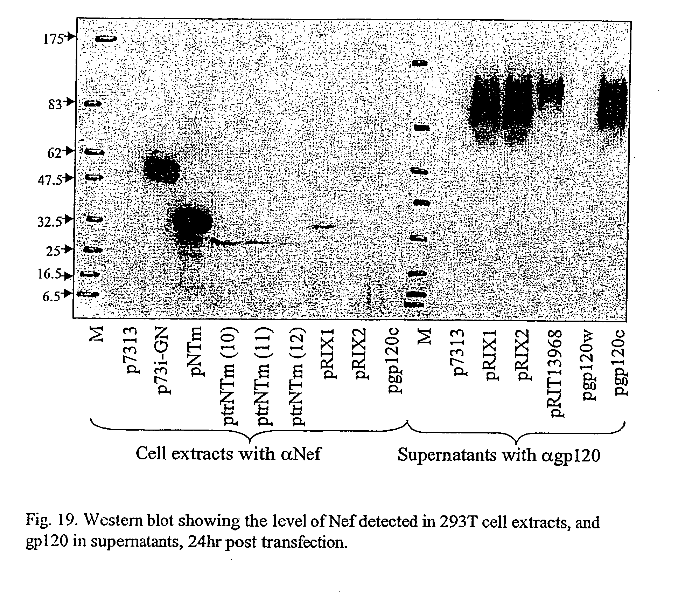

Expression vectors were constructed as described below. Endotoxin free plasmid preps were prepared and used to transfect subconfluent 293T cell monolayers in 24 well tissue culture plates with 1 µg of DNA using Lipofectamine 2000. Samples were harvested 24 hours post transfection and examined by Western Blot to assess expression levels. (Fig 18 and 19)

Codon optimisation of gp120 resulted in a substantial increase of REV independent expression.

### Generation of plasmids

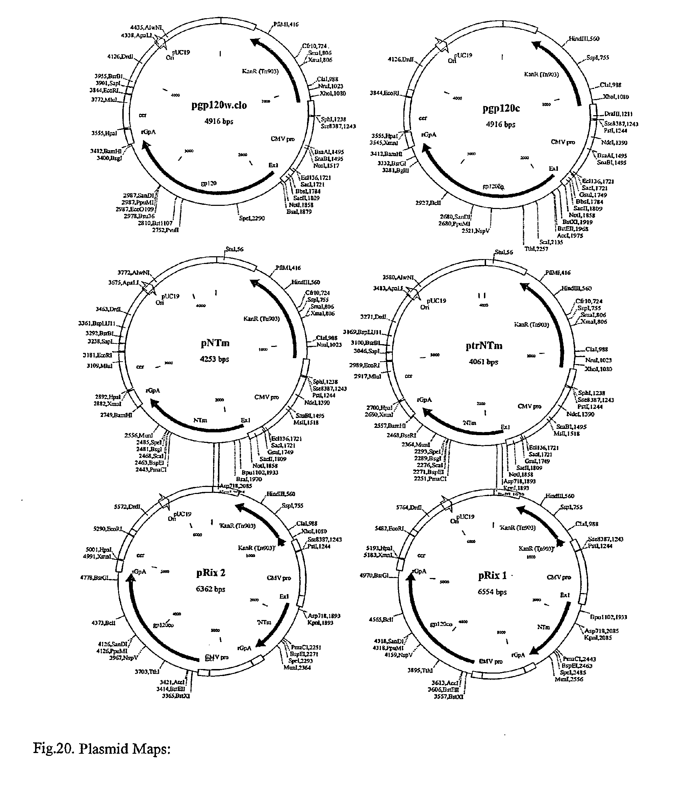

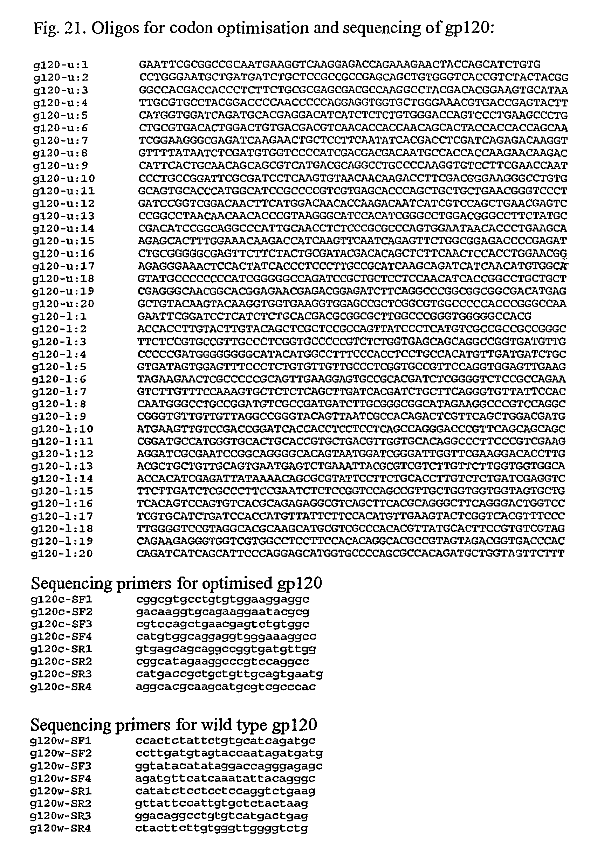

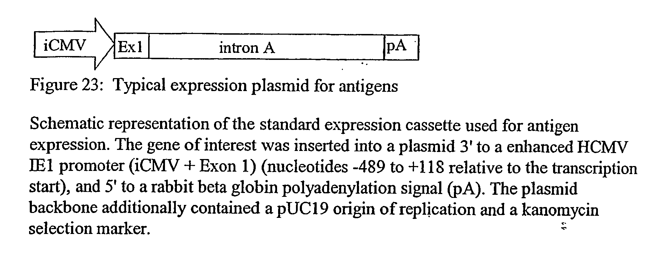

See also Figures 20 - 23

### gp120:

Both wild type and optimised gp120 were compared.

### Wild Type: (pgp120w)

PCR cloned from pRIT 13968 (see Example 11) using primers:
g120w-5' GAATTCGCGGCCGCAATGAAAGTGAAGGAGACCAG (Seq ID No: 32)
g120w-3' GAATTCGGATCCTTATCTCTGCACCAGTCTTC (Seq ID No: 33)

The gene was cloned into vector p7313-ie as a NotI-BamHI fragment and sequenced. A single base change (conservative) was found relative to the reference sequence, but this change was also found on sequencing pRIT 13968 (T1170C in gp120).

### Codon Optimised (pgp120c)

The gene sequence was based on the gp120 sequence from pRIT 13968. This has a RSCU value of 0.297. Optimisation was performed using SynGene 2d, resulting in a RSCU value of 0.749. The sequence was split into 40 overlapping oligonucleotide, PCR assembled and recovered using the end primers. The gene was cloned into vector p7313-ie as a NotI-BamHI fragment and sequenced. Restriction fragments from three initial clones were combined to generate a single correct clone.

### Nef/Tat (pNTm and ptrNTm)

The gene for the Nef/Tat fusion protein was provided in plasmid pRIT15244. The plasmid pRIT 15244 is identical to pRIT 14913 previously described except that the His tail has been deleted. The Tat in this plasmid contains three mutations as previously described. The fusion contains full length Nef which has an immune modulatory function (Collins and Baltimore (1999)) that may be abrogated by N-terminal truncation. Therefore constructs were generated for both full length Nef/mutant Tat(pNTm) and truncated Nef/mutant Tat(ptrNTm), in which the first 65 amino acids of Nef were removed. These sequences were PCR amplified from pRIT15244 using primers:
5'Nef: GAATTCGCGGCCGCCATGGGTGGCAAGTGGTCAAAAAG
5'trNef: GAATTCGCGGCCGCCATGGTGGGTTTTCCAGTCACACC
3'Tat: GAATTCGGATCCTTATTCCTTCGGGCCTGTCGGG (Seq ID Nos: 34, 35 and 36 respectively)

The genes were cloned into vector p7313-ie as NotI-BamHI fragments and sequenced.

**Dual expression vectors:** (pRIX1 and pRIX2)

The Nef/Tat and trNef/Tat expression cassettes were excised as ClaI-XnnI restriction fragments, and ligated into the ClaI and blunted Sse8387 I sites of the vector containing the codon optimised gp120 to provide single plasmids for expression of both proteins (pRIX1 and pRIX2 respectively).

### Composition of plasmid p7313-ie

The plasmid was constructed by replacing the beta-lactamase gene containing Eam1105I-Pstl fragment of pUC19 (available from Amersham Pharmacia Biotech UK Ltd., Amersham Place, Little Chalfont, Bucks, HP7 9NA) with an EcoRI fragment of pUC4K (Amersham-Pharmacia) containing the Kanamycin resistance gene, following blunt ending of both fragments using T4 DNA polymerase. The human Cytomegalovirus IE1 promoter /enhancer, Intron A, was derived from plasmid JW4303 obtained from Dr Harriet Robinson, University of Massachusetts, and inserted into the Sal1 site of pUC19 as a XhoI -Sal1 fragment, incorporating the bovine growth hormone polyadenylation signal. Deletion of the 5' SalI-BanI fragment from the promoter generated the minimal promoter used in the vector (WO00/23592-Powderject Vaccines Inc.). HBV Surface antigen 3'UTR was derived from Hepatitis B Virus, serotype adw, in the vector pAM6 (Moriarty et al., Proc.Natl.Acad.Sci. USA, 78, 2606-10,1981). pAM6 (pBR322 based vector) was obtained from the American Type Culture Collection, catalogue number ATCC 45020. The 3'UTR was inserted 5' to the polyadenylation signal as a 1.4kb BamHI fragment, blunt ended for insertion to remove the BamHI sites. In a series of steps (including digestion with *Bgl* II, Klenow polymerase treatment, digestion with *Bst*X I, digestion with *Nco* I, treatment with mung bean nuclease to remove overhang and further digestion with *BstX* I), modifications were made to the region between the 3'untranslated enhancer region of the HBV S gene and bGHpA signal to remove all open reading frames of greater than 5 codons between the X gene promoter and the bGHpA signal. This resulted in deletion of sequence encoding the translatable portion of the X protein (9 amino acids) and the X gene start codon. The bovine growth hormone polyadenylation signal was substituted with the rabbit beta globin polyadenylation signal. The 5'non-coding and coding sequences of the S antigen were excised and replaced with an oligonucleotide linker to provide multiple cloning sites as shown to produce plasmid p7313-PL.

Hind---NotI--    EcoRV-    -NdeI-    -BamHI
AGCTTGCGGCCGCTAGCGATATCGGTACCATATGTCGACGGATCC....
....ACGCCGGCGATCGCTATAGCCATGGTCTACAGCTGCCTAGGCCGG
          -NheI-      -KpnI-    -SalI-    ΔNotI
(Seq ID No: 37)

This polylinker was further extended by insertion of an additional oligonucleotide linker between the KpnI and SalI sites:

AspI-   -MunI-  NaeI-     NdeI--     BglII-
GTACCGGTCAATTGGCGCCGGCGCCATATGACGTCAGATCTG----
----GCCAGTTAACCGCGGCCGCGGTATACTGCAGTCTAGACAGCT
--AgeI-     -NarI--          AatII-       SalI
(Seq ID No: 38)

The ColE1 cer sequence was obtained from a subclone from plasmid pDAH212 from David Hodgeson (Warwick University) and amplified by PCR using primers to place EcoRI restriction sites at the ends of the sequence. The cer sequence was then inserted into the EcoRI site of p7313-PL to produce plasmid p7313-PLc. The sequence of the amplified cer was verified against the Genbank entry M11411.

The HBV 3'UTR sequence between the promoter and polyadenylation signal was removed by PCR amplification of the polyadenylation signal using primers: sense: CCATGGATCCGATCTTTTTCCCTCTGCC (Seq ID No: 39) antisense: GTTAGGGTGAAAAGCTTCCGAGTGAGAGACAC (Seq ID No: 40) The resulting product was cut with BamHI and XmnI and used to replace the corresponding fragment containing both the polyadenylation signal and the 3'UTR. The Intron A sequence was removed from the plasmid by PCR amplification of the CMV promoter/enhancer using primers:
sense: GCTAGCCTGCAGGCTGACCGCCCAACGAC (Seq ID No: 41) antisense: GTTCTCCATCGCGGCCGCACTCTTGGCACGGGG (Seq ID No: 42)

The resulting product was cut with Sse8387 I and NotI, and inserted back into the Sse8387 I and NotI sites of the parental vector.

### Example 16: IMMUNOGENICITY STUDIES

A mouse immunogenicity study may be performed to test the immunogenicity of gp120 and NefTat constructs delivered by particle mediated immunotherapeutic delivery (PMID) to show that the construct can generate an immune response *in vivo.* The DNA constructs may be used in combination with protein to determine whether there is an advantage of the combined approach compared to immunisation with the individual components. The aim is to detect and quantify cellular and humoral immune responses in mice following priming with protein and boosting with DNA (PMID) or priming with DNA (PMID) and boosting with protein.

The DNA is precipitated onto gold microparticles, which are used to coat the inner walls of a tefzel cartridge. PMID cartridges are prepared using a DNA loading rate of 2, which gives approximately 0.5 µg DNA per cartridge.

Mice are immunised by PMID on the abdomen by providing two shots containing gp120 or NetTat. Following a PMID boost spleens are removed and the spleen cells are used to determine immunogenicity to individual constructs. Responses are evaluated using the IFN-γ ELIspot assay. In this assay peptides from gp 120 and Nef are used to stimulate the spleen cells to secrete IFNγ which is captured by an antibody based detection system. The measurement of humoral responses to gp120 and Nef are measured by ELISA.

### gp120/NefTat Prime Boost Studies

The aim of the study is to detect and quantify cellular and humoral immune responses in mice following priming with protein and boosting with DNA (PMID) or priming with DNA (PMID) and boosting with protein.

### Cartridge Preparation

Gene gun cartridges are suitably prepared using a DNA loading rate (DLR) of 2, which will give approximately 0.5 µg DNA per cartridge.

### In vivo Immunogenicity

An experiment (Experiment I) may suitably be carried out as set out in the table below. The readouts for this experiment are carried out by IFN-γ ELIspot to Balb/c peptides from gp120 and Nef to measure the cellular responses and detection of antibodies to gp120 and Nef by ELISA for the humoral response.

### Experiment I

| **Group** | **Mouse strain** | **Prime** | **Boost** |
|---|---|---|---|
| A | Balb/c Female | PMID: gp120, 2 x 0.5 µg | PMID: gp120, 2 x 0.5 µg |
| B | Balb/c Female | PMID: NefTat, 2 x 0.5 µg | PMID: NefTat, 2 x 0.5 µg |
| C | Balb/c Female | PMID: Empty vector, 2 x 0.5 µg | MID: Empty vector, 2 x 0.5 µg |
| D | Balb/c Female | Protein: gp120 | Protein: gp120 |
| E | Balb/c Female | Protein: NefTat | Protein: NefTat |
| H | Balb/c Female | Protein: Irrelevant protein | Protein: Irrelevant protein |

| | | | |
|---|---|---|---|
| n=3 | | | |

A further experiment (Experiment II) may be carried out as follows:

### Experiment II

| **Group** | **Mouse strain** | **Prime** | **Boost** |
|---|---|---|---|
| A | Balb/c Female | PMID: gp120, 2 x 0.5 µg | Protein: gp120 |
| B | Balb/c Female | PMID: gp120, 2 x 0.5 µg | PMID: gp120, 2 x 0.5 µg |
| C | Balb/c Female | PMID: NefTat, 2 x 0.5 µg | Protein: NefTat |
| D | Balb/c Female | PMID: NefTat, 2 x 0.5 µg | PMID: NefTat, 2 x 0.5 µg |
| E | Balb/c Female | PMID: gp120, 2 x 0.5 µg Nefrat, 2 x 0.5 µg | Protein: gp120 + NefTat |
| F | Balb/c Female | PMID: gp120, 2 x 0.5 µg NefTat, 2 x 0.5 µg | PMID: gp120, 2 x 0.5 µg NefTat, 2 x 0.5 µg |
| G | Balb/c Female | PMID: Empty vector, 2 x 0.5 µg | Protein: Irrelevant protein |
| H | Balb/c Female | Protein: gp120 | PMID: gp120, 2 x 0.5 µg |
| I | Balb/c Female | Protein: gp120 | Protein: gp120 |
| J | Balb/c Female | Protein: NefTat | PMID: NefTat, 2 x 0.5 µg |
| K | Balb/c Female | Protein: NefTat | Protein: NefTat |
| L | Balb/c Female | Protein: gp120 + NefTat | PMID: gp120, 2 x 0.5 µg NefTat, 2 x 0.5 µg |
| M | Balb/c Female | Protein: gp120 + NefTat | Protein: gp120 + NefTat |
| N | Balb/c Female | Protein: Irrelevant protein | PMID: Empty vector, 2 x 0.5 µg |

| | | | |
|---|---|---|---|
| n=3 | | | |

### Sample protocol

Female Balb/c mice are used. All mice are pre-bled (tail vein) before the start of the experiment.

Groups of 3 mice/group are culled after the primary immunisation and spleens are collected for enumeration of IFN-γ -producing CD8 cells by ELIspot assay using gp120 and Nef K^{d}-restricted peptides.

Groups of 3 mice/group are culled at a minimum of two time points after the secondary immunisation and cardiac blood samples are collected. These are analysed for antibodies to gp120 and Nef using ELISA assays. Spleens are also be collected for enumeration of IFN-γ -producing CD8 cells by ELIspot assay using gp120 and Nef K^{d}-restricted peptides.

### Immunological Assays

### ELIspot assays

Cellular immune responses are detected using Interferon-γ ELIspot assays. Splenocytes will be isolated from 3 mice per group at each selected time point (one post primary and a minimum of two post-boost), and incubated overnight with peptides to known CD8 epitopes (restricted to a K^{d} (Balb/c) background) in plates coated with α--Interferon-γ. The splenocytes are lysed and plates developed using a secondary α-Interferon-γ antibody and biotin-streptavidin amplification system.

### ELISA assays

Humoral responses are detected using standard antibody ELISA assays. 96-well flat-bottomed microtitre plates will be coated with protein and blocked. Serial dilutions of serum collected prior to immunisation and after boost are collected and incubated in the plates. The plates are developed after incubation with an anti-mouse antibody. Responses to gp120, Nef and Tat are analysed.

### Example 17: IMMUNOGENICITY STUDIES

### Protocol

Cartridges were prepared for particle mediated delivery using a gene gun using standard methods. A DNA loading rate of 2 which will give approximately 0.5 µg DNA/cartridge was used.

F1 (C3H x Balb/c) mice were given a primary immunisation with either gp120 protein and NefTat in adjuvant (administered via the intramuscular route) or with gp120 DNA, codon optimised and cloned into vector p7313-ie as described in Example 15, and a vector expressing a Nef fusion protein (using particle mediated delivery wherein the DNA is coated onto gold beads). The mice were boosted 23 days later either with protein in adjuvant (administered via the intramuscular route) or with DNA (using particle mediated delivery wherein the DNA is coated onto gold beads). Mice were culled 5 days later and spleens were collected. The splenocytes were harvested by teasing out, erythocytes were lysed and splenocytes were washed and counted. Specialised ELIspot plates (coated with interferon-gamma capture antibody and blocked) were used. Splenocytes were transferred to these plates and incubated overnight at 37°C/5% CO₂ in the presence of medium control gp120 E7 peptide or various nef peptides. The splenocytes were lysed and the plate developed using standard procedures to demonstrate the number of interferon-gamma secreting cells present.

### Conclusion

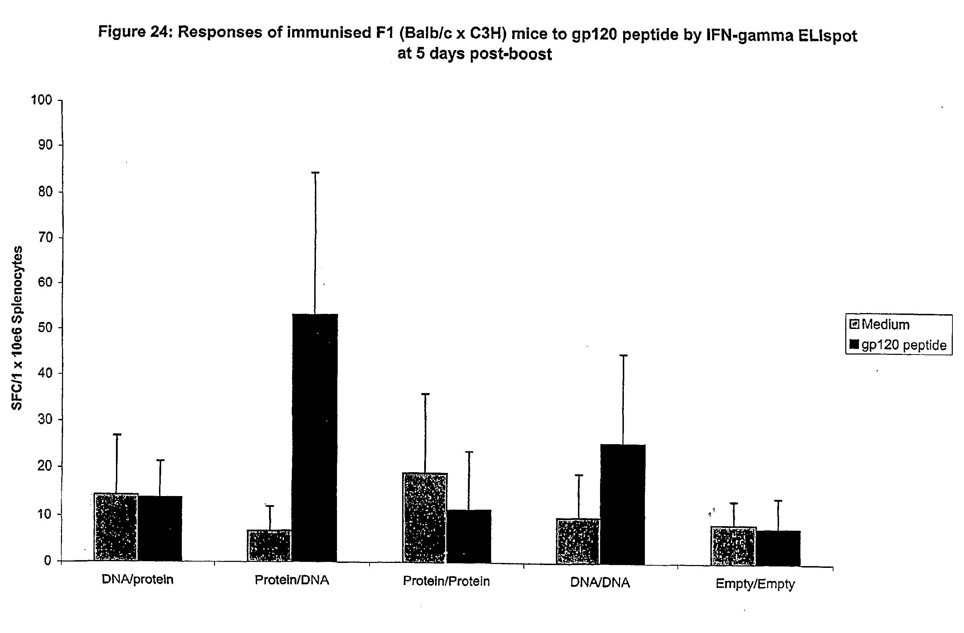

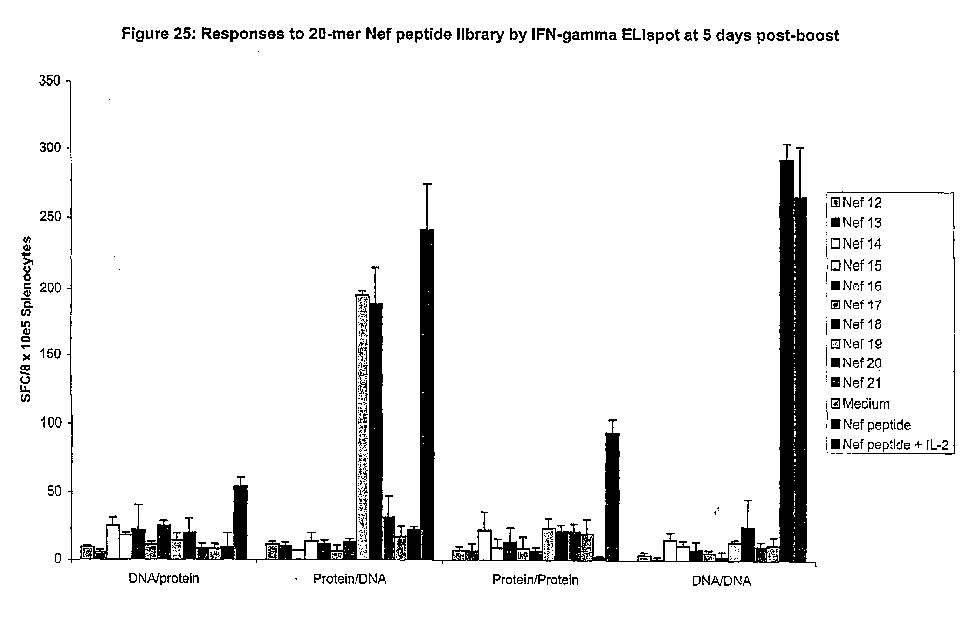

Although the ELIspot assay was carried out using a sub-optimal concentration of gp 120 E7 peptide, the results indicated that the most effective schedule was to prime mice with protein in adjuvant (administered i.m.) and then boost with DNA (administered by particle mediated delivery). See Figures 24. Similar results were obtained for nef, with the two peptides Nef 19 and 20 only being recognised by mice that had been primed with protein and boosted with DNA. The sequences for these two peptides are Nef 29: HIV-1 Bru (171-190) GMDDPEREVLEWRFDSRLAF (Seq ID No: 43) and Nef 20: HIV-1 Bru (181-200) EWRFDSRLAFHHVARELHPE (Seq ID No: 44). See Figure 25.

### Example 18: IMMUNOGENICITY STUDIES

### Protocol

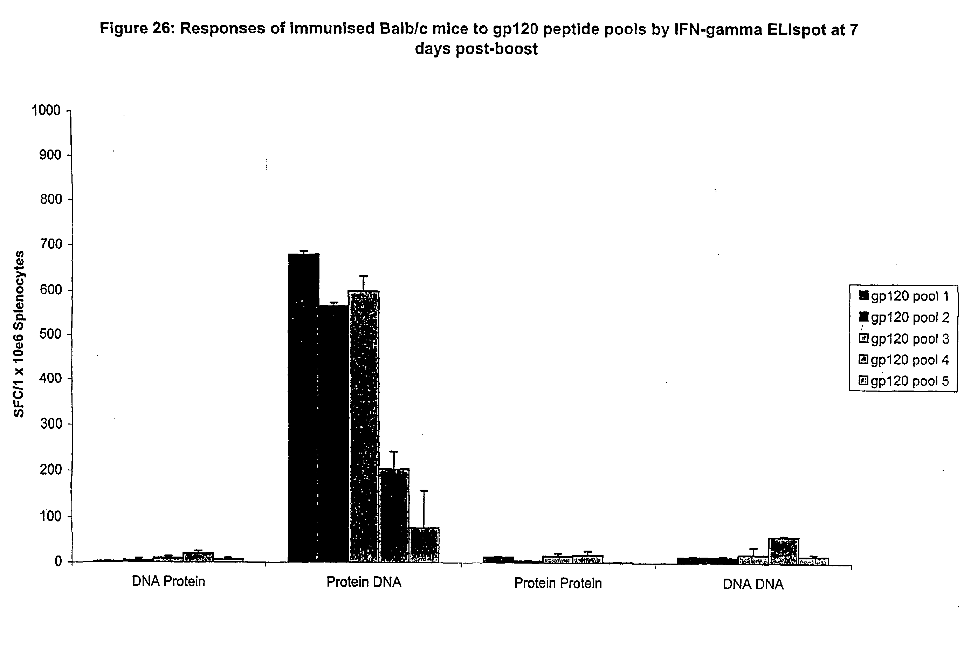

For PMID inununisations (DNA) cartridges were prepared using standard methods. A DNA loading rate of 2, which will give approximately 0.5 µg DNA/cartridge was used and each immunisation consisted of two shots. Protein was formulated in adjuvant comprising squalene/tocopherol/Tween 80/3D-MPL1QS21 just before use. Balb/c mice were given a primary immunisation of either gp120 protein in adjuvant (administered via the intramuscular route) or with gp120 codon optimised DNA prepared as described in Example 1 (using PM1D). The mice were boosted 21 days later with either protein in adjuvant (administered via the intramuscular route) or with DNA (using PMID). Mice were culled 7 days later and spleens were collected. The splenocytes were harvested by teasing out the spleen cells and erythrocytes were lysed. The splenocytes were washed and counted. Specialised ELIspot plates (coated with interferon-gamma capture antibody and blocked) were used. Splenocytes were transferred to these plates and incubated overnight at 37°C/5% CO₂ in the presence of pools of gp120 15-mer peptides. The splenocytes were lysed and the plate developed using standard procedures to demonstrate the number of interferon-gamma secreting cells present. Results are shown in Figure 26.

### Conclusion

Three pools of gp120 15-mer peptides were recognised by mice that had been primed with protein and boosted with DNA. Responses to these three pools of gp120 15-mers were not detected in animals that had been primed with DNA and boosted with protein or immunised twice with either protein or DNA.

## Claims

1. Use of
a) an HIV Tat protein or polynucleotide; or
b) an HIV Nef protein or polynucleotide; or
c) an HIV Tat protein or polynucleotide linked to an HIV Nef protein or polynucleotide;
and an HIV gp 120 protein or polynucleotide in the manufacture of a vaccine suitable for a prime-boost delivery for the prophylactic or therapeutic immunisation of humans against HIV, wherein the priming dose is the HIV gp 120 protein and HIV Tat protein and/or HIV Nef protein and the boosting dose is HIV gp 120 polynucleotide and HIV Tat polynucleotide and/or HIV Nef polynucleotide, and wherein the polynucleotide is delivered via a bombardment approach.

2. Use according to claim 1 wherein the bombardment approach comprises propelling particles into a target tissue of interest, typically the skin.

3. Use according to claim 2 wherein the particles are gold beads onto which the protein or polynucleotide has been coated.

4. Use according to claim 2 or claim 3 in which the particles are accelerated to high speed by a helium gas jet.

5. Use according to any one of claims 2 to 4 wherein the gold beads are 0.4-4.0 µm in diameter.

6. Use according to claim 5 wherein the gold beads are 0-6 to 2.0 µm in diameter.

7. Use according to any preceding claim wherein the polynucleotide encoding the nef, tat or gp120 is codon-optimised DNA.

8. Use according to any preceding claim in which the polynucleotide encoding the nef, tat and gp120 is present on a single vector.

9. Use according to claim 8 in which the vector comprises the nef, tat and gp 120 polynucleotides inserted 3' to an enhanced HCMV IE1 promoter.

10. Use according to claim 9 in which the vector is p7313 as shown in Figure 22.

11. Use according to any preceding claim in which additional regulatory or structural proteins of HIV such as Rev, Vif, Vpu, and Vpr or proteins derived from the HIV *gag or pol* genes (and/or polynucleotides encoding such regulatory or structural proteins) are included in the vaccine formulation.

12. Use according to any one of claims 1 to 11, wherein the protein is adjuvanted.

13. Use according to claim 12, wherein the adjuvant is a combination of QS21 and 3D-MPL.

## Patentansprüche

1. Verwendung von
a) einem HIV-Tat-Protein oder -Polynukleotid; oder
b) einem HIV-Nef-Protcin oder -Polynukleotid; oder
c) einem HIV-Tat-Protein oder -Polynukleotid, das mit einem HIV-Nef-Protein oder -Polynukleotid verknüpft ist;
und einem HIV-gp120-Protein oder -Polynukleotid bei der Herstellung eines Impfstoffs, der für eine Prime-Boost-Verabreichung geeignet ist, zur prophylaktischen oder therapeutischen Immunisierung von Menschen gegen HIV, wobei die Priming-Dosis das HIV-gp120-Protein und HIV-Tat-Protein und/oder HIV-Nef-Protein ist und die Booster-Ddosis das HIV-gp120-Polynukleotid und HIV-Tat-Polynukleotid und/oder HIV-Nef-Polynukleotid ist, und wobei das Polynukleotid durch ein Beschussverfahren zugeführt wird.

2. Verwendung gemäß Anspruch 1, wobei das Beschussverfahren das Treiben von Partikeln in ein Zie-gewebe von Interesse, typischerweise die Haut, umfasst.

3. Verwendung gemäß Anspruch 2, wobei die Partikel Goldperlen sind, auf denen das Protein oder das Polynukleotid beschichtet worden ist.

4. Verwendung gemäß Anspruch 2 oder Anspruch 3, bei der die Partikel durch einen Heliumgasstrahl auf Hoehgeschwindigkeit beschleunigt werden.

5. Verwendung gemäß einem der Ansprüche 2 bis 4, wobei die Goldperlen einen Durchmesser von 0,4 bis 4,0 µm aufweisen.

6. Verwendung gemäß Anspruch 5, wobei die Goldperlen einen Durchmesser von 0,6 bis 2,0 µm aufweisen.

7. Verwendung gemäß einem der vorangehenden Ansprüche, wobei das Polynukleotid, das für das nef, tat oder gp120 kodiert, Kodon-optimierte DNA ist.

8. Verwendung gemäß einem der vorangehenden Ansprüche, bei der das Polynukleotid, das für das nef, tat und gp120 kodiert, auf einem einzelnen Vektor vorliegt.

9. Verwendung gemäß Anspruch 8, bei der der Vektor das nef-, tat- und gp120-Polynukleotid 3' zu einem verstärkten HCMV-IE1-Promotor eingefügt umfasst.

10. Verwendung gemäß Anspruch 9, bei der der Vektor p7313 ist, wie in Figur 22 gezeigt.

11. Verwendung gemäß einem der vorangehenden Ansprüche, bei der zusätzliche Regulator- oder Strukturproteine von HIV wie Rev, Vif, Vpu und Vpr oder Proteine, die von den HIV-gag- oder -pol-Genen abgeleitet sind (und/oder Polynukleotide, die für solche Regulator- oder Strukturproteine kodieren), in der Impfstofifformulierung enthalten sind.

12. Verwendung gemäß einem der Ansprüche 1 bis 11, wobei das Protein adjuvantiert ist.

13. Verwendung gemäß Anspruch 12, wobei das Adjuvans eine Kombination aus QS21 und 3D-MPL ist.

## Revendications

1. Utilisation :
a) d'une protéine ou d'un polynucléotide Tat du VIH; ou
b) d'une protéide ou d'un polynucléotide Nef du VIH; ou
c) d'une protéine ou d'un polynucleotide Tat du VIH lié à une protéine ou à un polynucléotide Nef du VIH;
et d'une protéine ou d'un polynucléotide gp120 du VIH dans l'élaboration d'un vaccin pouvant être administré par une primovaccination suivie d'un rappel (prime-boost) pour l'immunisation prophylactique ou thérapeutique d'êtres humains contre le VIH, dans laquelle la dose de primovaccination est la protéine gp120 du VIH et la protéine Tat du VIH et/ou la protéine Nef du VIH et la dose de rappel est un polynucleotide gp12G du VIH et un polynucléotide Tat du VIH et/ou un polynucléotide Nef du VIH, et dans laquelle le polynucleotide est administré par une technique de bombardement.

2. Utilisation selon la revendication 1, dans laquelle la technique de bombardement consiste à projeter des particules dans un tissu cible d'intérêt, généralement la peau.

3. Utilisation selon la revendication 2, dans laquelle les particules sont des billes d'or sur lesquelles la protéine ou le polynucléotide ont été appliqués par enrobage.

4. Utilisation selon la revendication 2 ou la revendication 3, dans laquelle les particules sont accélérées jusqu'à une vitesse élevée par un jet d'hélium gazeux.

5. Utilisation selon l'une quelconque des revendications 2 à 4, dans laquelle les billes d'or ont un diamètre de 0,4 à 4,0 µm.

6. Utilisation selon la revendication 5, dans laquelle les billes d'or ont un diamètre de 0,6 à 2,0 µm.

7. Utilisation selon l'une quelconque des revendications précédentes, dans laquelle le polynucléotide codant pour nef, tat ou gp120 est un ADN à codons optimisés.

8. Utilisation selon l'une quelconque des revendications précédentes, dans laquelle le polynucléotide codant pour nef, tat et gp120 se trouve sur un seul vecteur.

9. Utilisation selon la revendication 8, dans laquelle le vecteur comprend les polynucleotides nef, tat et gp120 insérés en 3' d'un promoteur amplificateur IE1 du CMVH.

10. Utilisation selon la revendication 9, dans laquelle le vecteur est p73313 représenté sur la figure 22.

11. Utilisation selon l'une quelconque des revendications précédentes, dans laquelle d'autres protéines régulatrices ou structurales du VIH telles que Rev, Vif, Vpu et Vpr ou des protéines dérivées des gènes gag ou pol du VIH (et/ou des polynucleotides codant pour ces protéines régulatrices ou structurales) sont incluses dans la formulation du vaccin.

12. Utilisation selon l'une quelconque des revendications 1 à 11, dans laquelle la protéine est adjuvée.

13. Utilisation selon la revendication 12, dans laquelle l'adjuvant est une combinaison de QS21 et de 3D-MPL.
